# EUROPEAN PATENT APPLICATION

(11) **EP 4 338 757 A1**
(43) Date of publication of application: **20.03.2024**
(21) Application number: 22195665.9
(22) Date of filing: 14.09.2022
(51) Int. Cl.: A61K 51/04, A61K 103/00, C07H 15/04

(54) **NEW PET TRACER FOR IMAGING OF THE FUNCTIONAL LIVER RESERVE**

(71) Applicant: Medizinische Universität Innsbruck, 6020 Innsbruck (AT)
(72) Inventor: HAUBNER, Roland, 6020 Innsbruck (AT); ZIERKE, Maximilian Alexander, 6020 Innsbruck (AT); RANGGER, Christine, 6020 Innsbruck (AT)
(74) Representative: Grünecker Patent- und Rechtsanwälte PartG mbB

(57) **Abstract**

The present invention relates to compounds of formula (I)

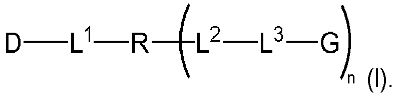

In the compounds of formula (I), D is a chelating group, a prosthetic group or a [¹⁸F]F accepting group; -L'-R is absent or L¹ is a linker and R is a multivalent group; L² is a linker; L³ is absent or a linker; G is Gal, GaINAc, or Lac; and n is 3 or 4.

The compounds are useful as PET (positron emission tomography) tracers.

## Description

### Field of the Invention

The present invention relates to new and inventive compounds, especially compounds useful as PET (positron emission tomography) tracer. The present invention further relates to a pharmaceutical or diagnostic composition comprising such a compound. The present invention further relates to a method of synthesizing the compound. The present invention further relates to a method of imaging hepatocytes, comprising a) contacting the hepatocyte with such a compound and b) visualizing the compound that is in contact with the hepatocyte. Also, the present invention relates to a method of determining functional hepatic reserve, comprising a) contacting the liver with such a compound, and b) visualizing the compound that is in contact with the liver. The present invention further relates to the use of the compound for imaging hepatocytes as well as to the use for determining functional hepatic reserve.

### Background of the Invention

The asialoglycoprotein receptor (ASGR) is a c-type lectin mainly expressed on the basolateral side of hepatocytes, where up to 500.000 receptors per cell can be found (D'Souza AA et al. J Control Release 2015;203:126-39) . In contrast, in the remainder of the body expression is low making it a promising target for e.g. drug delivery into hepatocytes. The main physiological function of the ASGR is to maintain serum glycoprotein homeostasis by clearing of desialylated glycoproteins bearing D-galactose (Gal) or N-acetylgalactosamine (GalNAc) as terminal carbohydrates. Therefore, these glycoproteins are internalized via receptor-mediated endocytosis. This is initiated by binding of the glycoprotein to the ASGR, followed by the migration of the receptor-ligand complex to active internalization sites (clathrin coated pits) where corresponding vesicles are formed leading to the internalization of the complex. Subsequently, in the cell, the glycoprotein is degraded in the lysosome and the ASGR is recycled (Stockert RJ. et al. Physiol Rev 1995;75:591-609; de Graaf W et al. J Nucl Med 2010;51:742-52). The ASGR consists of two homologous subunits H1 and H2, which are responsible for Gal/GalNAc recognition (Lee YC et al. J Biol Chem 1983;258:199-202). Carbohydrate binding was elucidated by X-ray structure analysis of the carbohydrate recognition domain (CRD) of the H1 subunit (Meier M et al. J Mol Biol 2000;300:857-65).

Non-invasive methods allowing quantitative determination of the functional liver mass are of great interest for patient management in a diversity of clinical settings comprising liver surgery and liver transplantation (de Graaf W et al. J Nucl Med 2010;51:742-52; Hoekstra LT et al. Ann Surg 2013;257:27-36; Kaibori M et al. Ann Nucl Med 2011;25:593-602) as well as diagnosis (Virgolini I etal.. Br J Cancer 1990;61:937-41; Kurtaran A et al. J Nucl Med 1995;36:1875-81) and treatment monitoring (Virgolini I et al. Br J Cancer 1993;68:549-54) of cancer. Additionally, it has been revealed that the evaluation of remnant liver function can help to discriminate different stages of alcoholic liver cirrhosis (Virgolini I et al. Nucl Med Commun 1991;12:507-17) and could be used to differentiate areas of steatosis, fibrosis and cholestasis (Bennink RJ et al. Semin Nucl Med 2012;42:124-37) . Moreover, control of liver status before and during peptide receptor radionuclide therapy (PRRT) (Mansi L et al. Eur J Nucl Med Mol Imaging 2011;38:605-12)could lead to an optimized patient management. Further, patients who are potentially suitable for selective internal radiation therapy (SIRT) (D'Arienzo M et al. Nucl Med Commun 2012;33:633-40) may also benefit from such a diagnostic method, allowing stratifying patients according to their peri-interventional risk.

Due to the restricted expression, the ASGR is an optimal target structure for non-invasive monitoring of the liver function. [^{99m}Tc]Tc-diethylenetriamine-pentaacetic acid galactosyl human serum albumin (^{99m}Tc-GSA) (Kokudo N et al. Nucl Med Biol 2003;30:845-9) has been developed to image ASGR expression using single photon emission tomography (SPECT). It has been shown that ^{99m}Tc-GSA and dynamic SPECT allows estimation of regional hepatic function based on the determination of the ASGR density (Kudo M et al. Hepatology 1993;17:814-9; Vera DR et al. J Nucl Med 1996;37:160-4). To combine the superior performance of positron emission tomography (PET) compared with SPECT concerning imaging resolution and quantifying properties with the excellent properties of GSA in ASGR targeting we developed a [⁶⁸Ga]Ga-labelled analogue (Haubner R et al. Eur J Nucl Med Mol Imaging 2016;43:2005-13). [⁶⁸Ga]Ga-DTPA-GSA showed comparable targeting properties as found for ^{99m}Tc-GSA but lacks high metabolic stability. By replacing DTPA with 2-S-(4-isothiocyanatobenzyl)-1,4,7-triazacyclononane-1,4,7-triacetic acid (p-NCS-Bn-NOTA) [⁶⁸Ga]Ga-NOTA-GSA was developed which demonstrated the desired high metabolic stability, which was even higher as for the original ^{99m}Tc-labelled DTPA-GSA, allowing the use of pharmacokinetic modeling approaches for more detailed quantification (Haubner R et al. Mol Imaging Biol 2017;19:723-30). Moreover, it confirms the superiority of PET, which includes the possibility of time-resolved 3D volume determination allowing accurate quantification of the tracer dynamics throughout the entire liver and enabling the reader to delineate exactly the volumes of interest and move from a rough estimation of liver function to a spatially resolved quantification. Despite the good imaging performance of human GSA-based radiotracers the translation of this class of compounds into clinical practice is limited due to regulations of biological products isolated from human material. The requirements in the production of a GMP-compliant labelling precursor, which are essential for use in humans, increased during the last years. Thus, despite the good imaging performance, the translation of this human serum albumin based labelling precursor into clinical practice was not possible indicating the need of radiolabelled low molecular weight, synthetic derivatives as galactose carrier.

Therefore, particular research interest has arisen in the development of galactose based synthetic small molecule radiotracers.

### Summary of the Invention

It is the objective of the present invention to provide low molecular weight PET tracer for the non-invasive determination of the asialoglycoprotein receptor expression, and in particular for determining functional hepatic reserve.

This problem is solved according to the present invention by the following embodiments and aspects of the invention.

In a first aspect, the present invention relates to a compound according to formula (I) 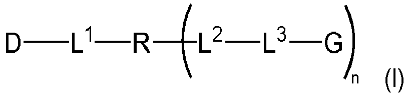 wherein D, R, L¹-L³, G and n are defined as disclosed below.

In a further aspect, the present invention relates to a pharmaceutical composition comprising the compound according to formula (I) and a pharmaceutically acceptable excipient.

In a further aspect, the present invention relates to a method of producing the compound according to formula (I), the method comprising synthesizing the compound.

In a further aspect, the present invention relates to a pharmaceutical or diagnostic composition comprising the compound according to formula (I) and at least one additive.

In a further aspect, the present invention relates to a method of imaging hepatocytes, comprising a) contacting the hepatocyte with a compound according to formula (I), and b) visualizing the compound that is in contact with the hepatocyte.

In a further aspect, the present invention relates to a method of determining functional hepatic reserve, comprising a) contacting the liver with the compound according to formula (I), and b) visualizing the compound that is in contact with the liver.

In a further aspect, the present invention relates to the use of the compound according to formula (I) for imaging hepatocytes.

In a further aspect, the present invention relates to the use of the compound according to formula (I) for determining functional hepatic reserve.

### Brief Description of the Drawings

The invention will be described in more detail in the following section and illustrated in accompanying figures.
**Figure 1** shows a radio-HPLC of [⁶⁸Ga]Ga-NODAGA-TriGalactan. Column: Reprosil Pur C₁₈ AQ 150 x 4.6 mm, Gradient: 5-60 % B in 15 min. t_{R} = 7.19 min.
**Figure 2** shows biodistribution data (% ID/g) of [⁶⁸Ga]Ga-NODAGA-TriGalactan (n = 3) and [^{99m}Tc]^{99m}Tc-GSA (n = 3) in healthy BALB/c mice at 10, 30 and 60 min p.i.
**Figure 3** shows the liver-to-organ ratios for [⁶⁸Ga]Ga-NODAGA-TriGalactan and [^{99m}Tc]^{99m}Tc-GSA 10, 30 and 60 min p.i.
**Figure 4** shows biodistribution data (% ID/g) of [⁶⁸Ga]Ga-NODAGA-TriGalactan (n = 3) in healthy BALB/c mice at 30 min p.i. and at 30 min blocked with 277 mM galactose.
**Figure 5** shows a radio-HPLC of [⁶⁸Ga]Ga-TRAP-Galactan. Column: Reprosil Pur C₁₈ AQ 150 x 4.6 mm, Gradient: 5-15 % B in 15 min. t_{R} = 12.7 and 13.3 min.
**Figure 6** shows a radio-HPLC of [⁶⁸Ga]Ga-Galacto-TRAP. Column: Reprosil Pur C₁₈ AQ 150 x 4.6 mm, Gradient: 1-10 % B in 15 min. t_{R} = 8.5 and 9.8 min.
**Figure 7** shows biodistribution data (% ID/g) of [⁶⁸Ga]Ga-TRAP-Galactan (n = 3) and [⁶⁸Ga]Ga-Galacto-TRAP (n = 3) in healthy BALB/c mice at 10, 30 and 60 min p.i.

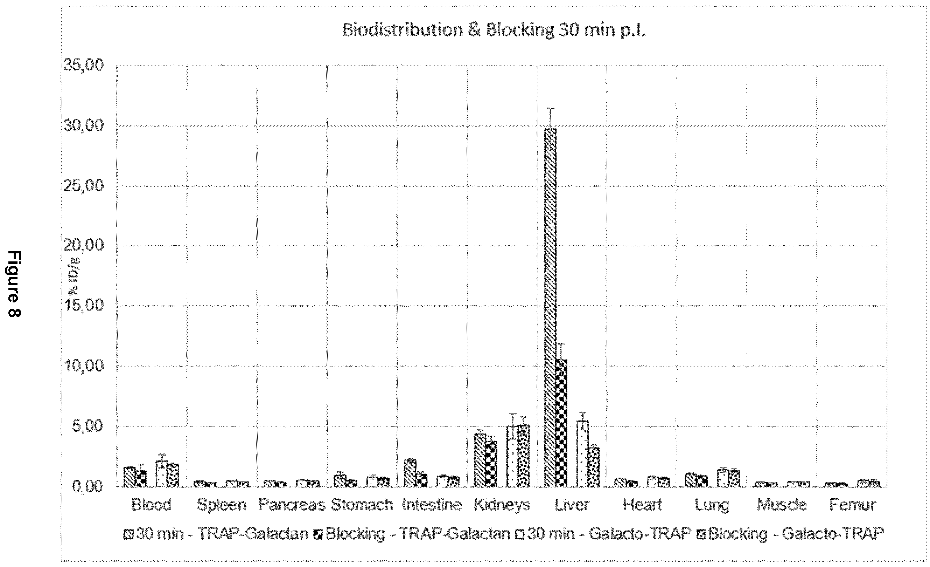

**Figure 8** shows biodistribution data (% ID/g) of [⁶⁸Ga]Ga-TRAP-Galactan (n = 3) and [⁶⁸Ga]Ga-Galacto-TRAP in healthy BALB/c mice at 30 min p.i. and at 30 min blocked with 277 mM galactose.
**Figure 9** shows liver-to-organ ratios for [⁶⁸Ga]Ga-TRAP-Galactan (n = 3) and [⁶⁸Ga]Ga-Galacto-TRAP (n = 3) 10, 30 and 60 min p.i.

### Detailed Description of the Invention

All publications, including but not limited to patents, patent applications and scientific publications, cited in this description are herein incorporated by reference for all purposes as if each individual publication were specifically and individually indicated to be incorporated by reference.

The use of the term "comprising" as well as other grammatical forms such as "comprises" and "comprised" is not limiting. The terms "comprising", "comprises" and "comprised" should be understood as referring to an open-ended description of an embodiment of the present invention that may, but does not have to, include additional technical features in addition to the explicitly stated technical features. In the same sense the term "involving" as well as other respective grammatical forms such as "involves" and "involved" is not limiting. The same applies for the term "including" and other grammatical forms such as "includes" and "included". Section headings throughout the description are for organizational purposes only. In particular, they are not intended as limiting for various aspects and embodiments described therein, and it is to be understood that aspects and embodiments (and features therein) described under one subheading may be freely combined with aspects and embodiments (and features therein) described under another subheading. Further, the terms "comprising", "involving" and "including", and any grammatical forms thereof, are not to be interpreted to exclusively refer to embodiments that include additional features to those explicitly recited. These terms equally refer to embodiments that consist of only those features that are explicitly mentioned.

The skilled person understands that the present invention is not limited to the embodiments explicitly mentioned above and below, but also includes combinations of aspects, embodiments or features that are not explicitly discussed herein, but can be implicitly derived.

The present invention relates to a compound of formula (I), or a pharmaceutically acceptable salt thereof, 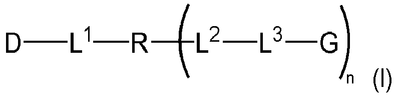 wherein
D is a chelating group or a prosthetic group or a [¹⁸F]F accepting group;
-L¹-R is absent or
L¹ is a linker selected from the group consisting of *-N(H)-(CH₂)ₓ-C(O)-, aspartic acid, glutamic acid, asparagine, glutamine, lysine, *-N(H)-(CH₂-CH₂-O)_{y}-(CH₂)_{z}-C(O)-, 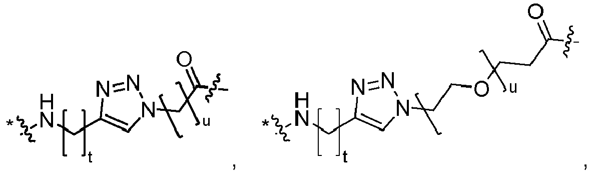 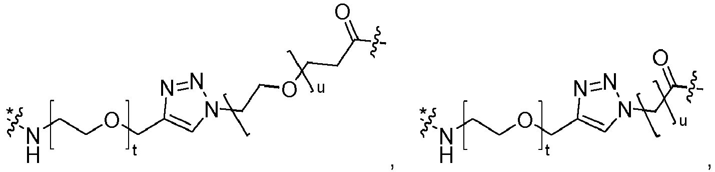 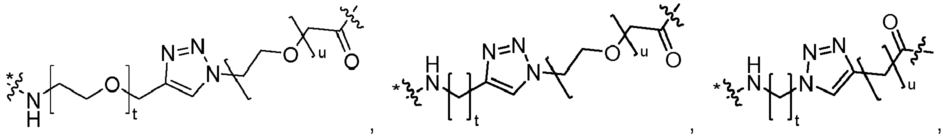 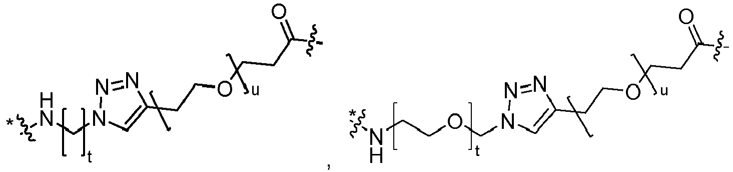 and 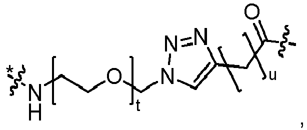 wherein* designates the attachment point to D, and
R is a multivalent group of the following formula 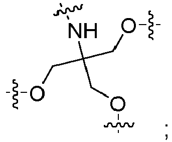
L² is a linker selected from the group consisting of 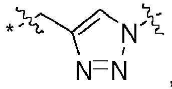 , *-CH₂-C(O)-NH-, *-CH₂- 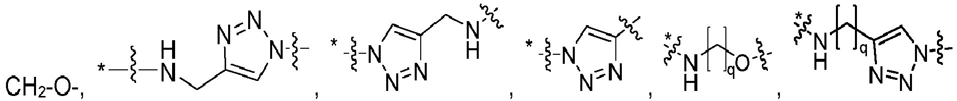 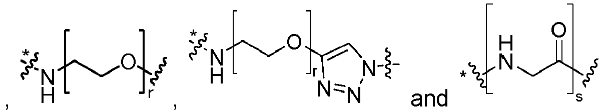 wherein * designates
   i) the attachment point to D, if -L¹-R is absent, or
   ii) the attachment point to R, if -L¹-R is present;
L³ is absent or a linker selected from the group consisting of ^{#}-(CH₂-CH₃-O)ₘ-CH₂-CH₂-, ^{#}-(CH₂)ₘ-, and 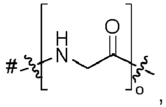 wherein ^{#} designates the attachment point to L²;
G is Gal, GalNAc, or Lac;
m is independently selected from 1-36;
n is 3-4, with the proviso that n is 3 if -L¹-R is present;
o is 3-6;
q is independently selected from 1-16;
r is independently selected from 1-36;
s is 1-10;
t is independently selected from 1-8;
u is independently selected from 1-8;
x is 1-16;
y is 1-36; and
z is 1-2.

In some embodiments, D is a chelating group selected from the group consisting of NODAGA, NOTA, DOTAGA, DO3A, HBED, NCS-DTPA, p-NCS-DFO, FusC, DafC, CNAAZTA, NOPO, TRAP, DOTPI, DOTAZA, sacrophagine-based chelators, TE2A derivatives such as CB-TE2A. Particularly preferred are NODAGA and TRAP. In particular, NODAGA is preferred if -L¹-R is present and TRAP is preferred if -L¹-R is absent. It is preferred that NODAGA chelates [⁶⁸Ga]Ga or [¹⁸F]AlF. It is preferred that TRAP chelates [⁶⁸Ga]Ga or [¹⁸F]AlF.

In some embodiments, the moiety -L¹-R is absent. If-L¹-R is absent, L² is directly bound to D.

In some embodiments, the moiety -L¹-R is present. Preferably, the linker L¹ binds to the multivalent group R through the amino functionality of

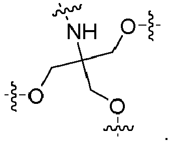

In some embodiments, -L¹-R is present and D is a chelating group selected from the group consisting of 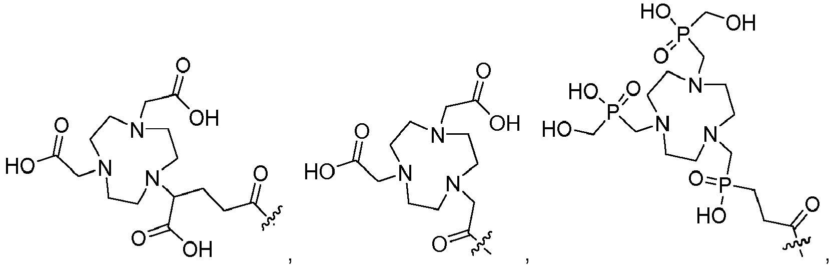 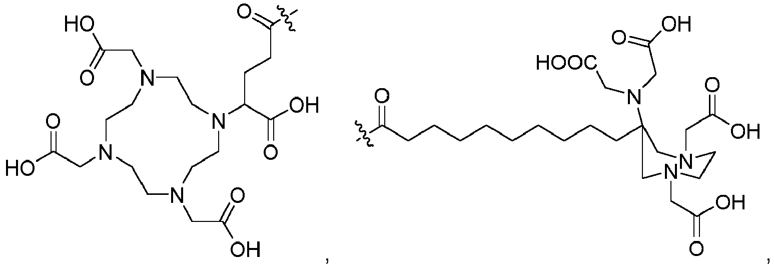 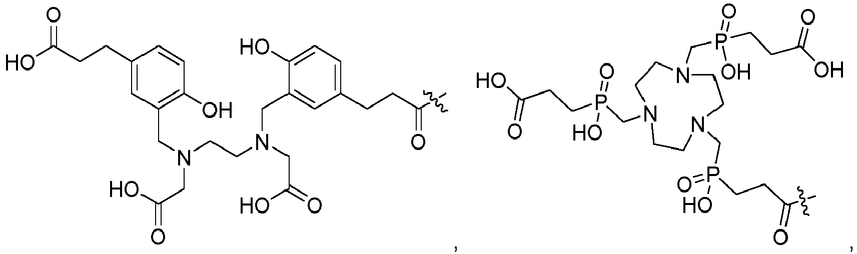 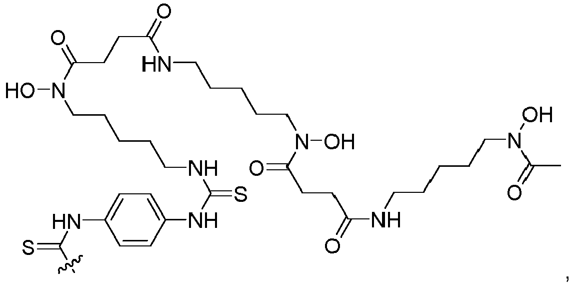 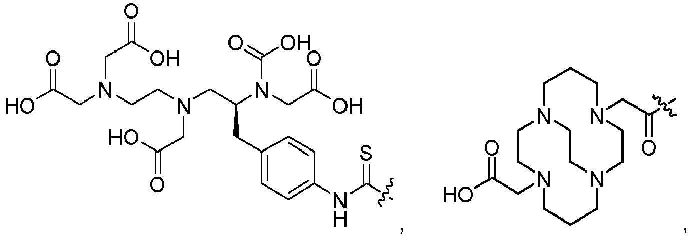 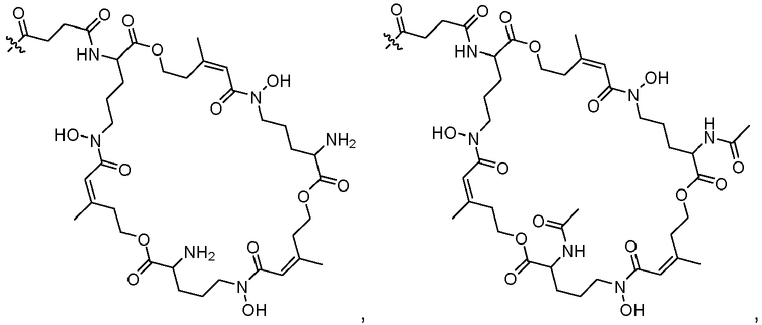 and

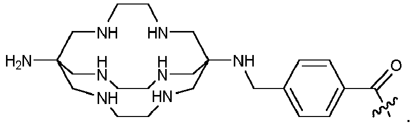

In some embodiments, -L¹-R is present and the chelator D is selected from

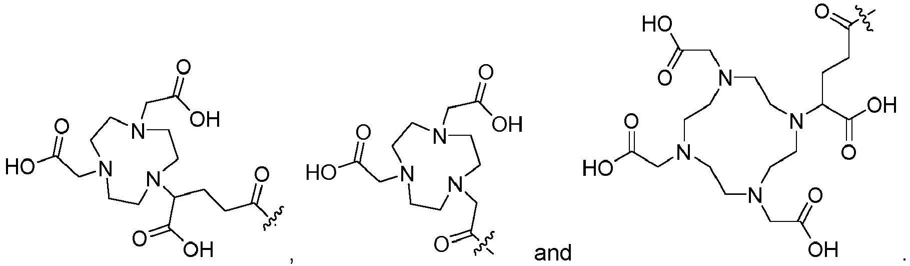

Most preferably, if -L¹-R is present in the compound according to formula (I), the chelator D is

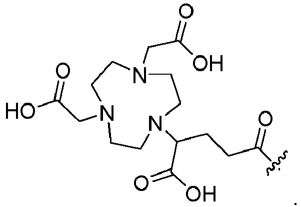

In some embodiments, L¹ is a linker selected from the group consisting of *-N(H)-(CH₂)ₓ-C(O)-, aspartic acid, glutamic acid, asparagine, glutamine, lysine, *-N(H)-(CH₂-CH₂-O)_{y}-(CH₂)_{z}-C(O)- 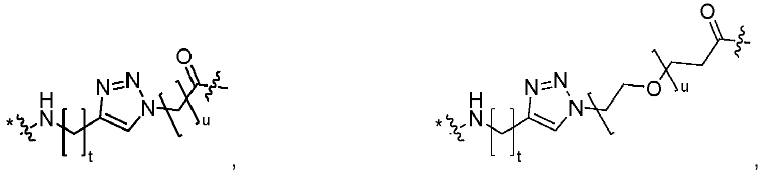 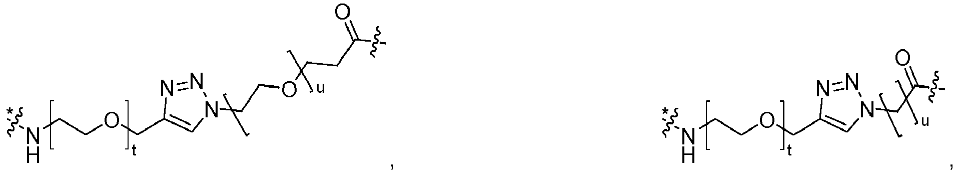 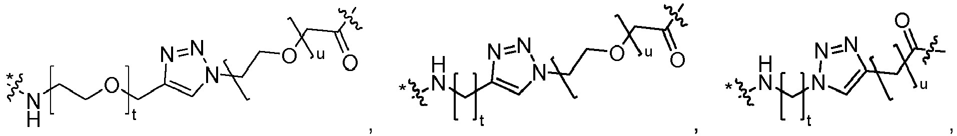 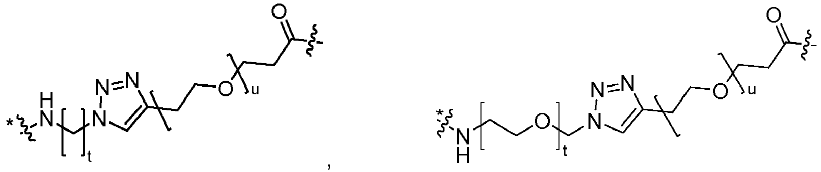 and 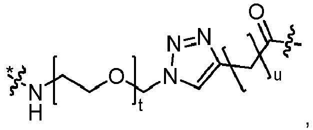 wherein* designates the attachment point to D.

In preferred embodiments, L¹ is *-N(H)-(CH₂)ₓ-C(O)-, and * designates the attachment point to D. The index x in *-N(H)-(CH₂)ₓC(O)- represents an integer from 1-16. In preferred embodiments, x is 2-10, 2-8 or 2-6. Most preferably, x is 3.

In some embodiments, -L¹-R is present and L² is selected from 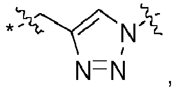 *-CH₂-C(O)-NH-, and *-CH₂-CH₂-O-, wherein * designates the attachment point to R.

In a more preferred embodiment, -L¹-R is present and L² is 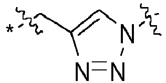 wherein * designates the attachment point to R.

In some embodiments, -L¹-R is present and L³ is selected from ^{#}-(CH₂-CH₃-O)ₘ-CH₂-CH₂-, ^{#}-(CH₂)ₘ-, and 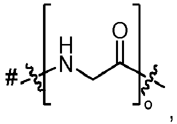 wherein ^{#} designates the attachment point to L². In more preferred embodiments, -L¹-R is present and L³ is ^{#}-(CH₂-CH₃-O)ₘ-CH₂-CH₂-, and ^{#} designates the attachment point to L². The index m in ^{#}-(CH₂-CH₂-O)ₘ-CH₂-CH₂- represents an integer from 1-36, preferably from 2-24, 2-18, 2-12 or 2-6. Most preferably, m is 2. ^{#}-(CH₂-CH₂-O)ₘ-CH₂-CH₂- for m=2 is ^{#}-CH₂-CH₂-O-CH₂-CH₂-O-CH₂-CH₂-.

In some embodiments, -L¹-R is present and the linker unit -L²-L³- connecting the sugar moiety G with the hydroxy functionalities of the multivalent group R has a length of about 10-30 Å, preferably about 14-20 Å. For instance, the length of -L²-L³- can be about 11 Å, 12 Å, 13 Å, 14 Å, 15 Å, 16 Å, 17 Å, 18 Å, 19 Å, 20 Å, 21 Å, 22 Å, 23 Å, 24 Å, 25 Å, 26 Å, 27 Å, 28 Å or 29 Å.

In a preferred embodiment, the compound is

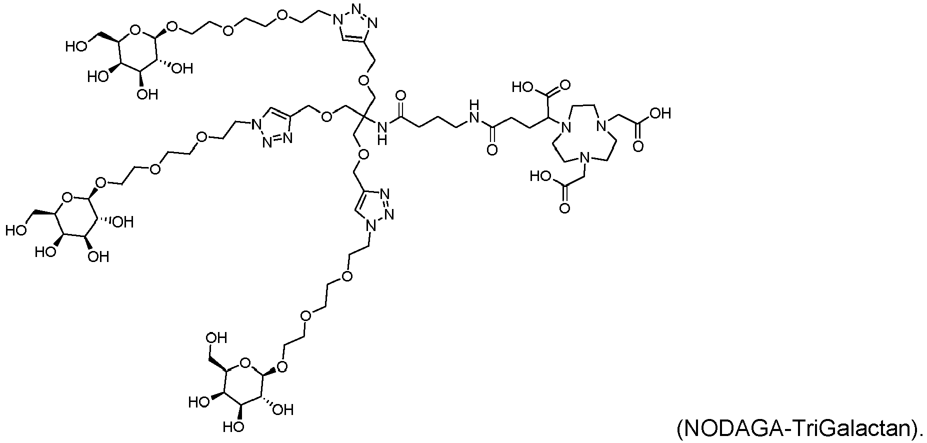

In some embodiments, the moiety -L¹-R is absent; the compounds of the present invention are then represented by structural formula (II)

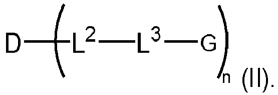

Compounds comprised by structural formula (II) have a group D as the central functionality to which sugar moieties G are attached via the linker group L²-L³. Preferably, D is a chelating group.

In some embodiments, -L¹-R is absent and D is a chelating group selected from the group consisting of 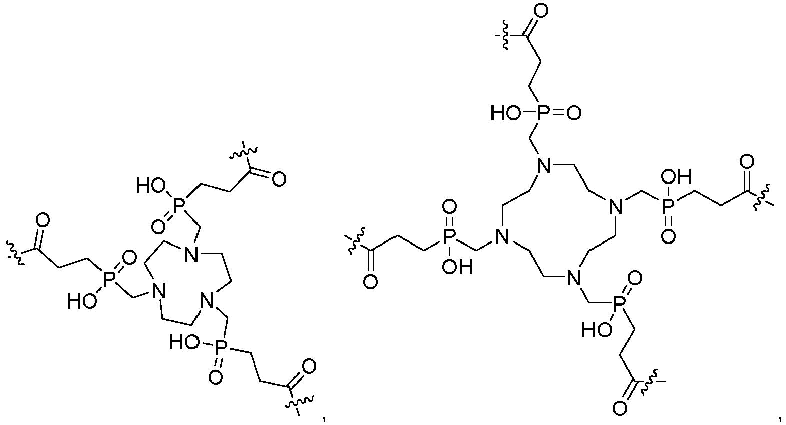 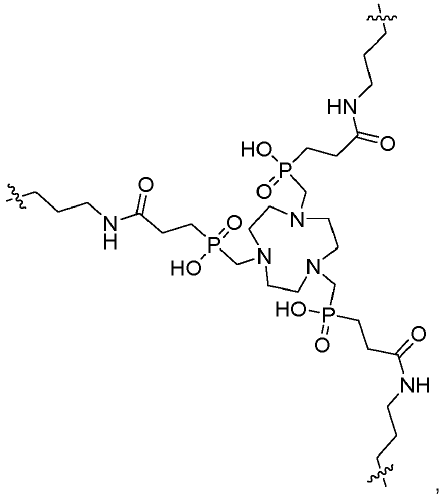 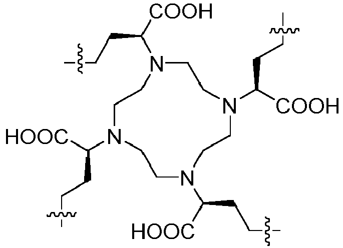 and

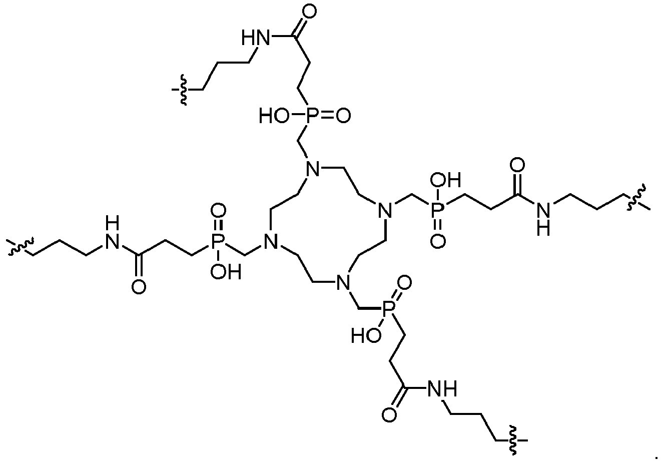

In more preferred embodiments, -L¹-R is absent and the chelator D is 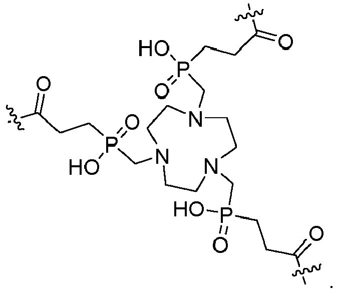 In some embodiments, -L¹-R is absent and L² is selected from 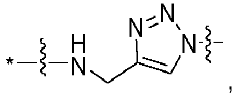 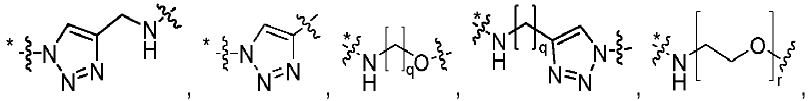 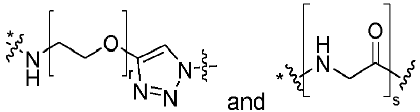 wherein * designates the attachment point to D.

In more preferred embodiments, -L¹-R is absent and L² is 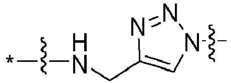 wherein * designates the attachment point to D. The index q ranges from 1-16, preferably from 1-12, 1-6, 1-4 or 1-3. In a more preferred embodiment, the index q is 1 or 2. The index r ranges from 1-36, preferably from 1-30, 1-24, 1-18, 1-12, 1-10, 1-8, 1-6, 1-4 or 1-3. In a more preferred embodiment, the index r is 1 or 2.The index s ranges from 1-10, preferably from 1-8, 1-6, 1-4 or 1-3. In a more preferred embodiment, the index s is 1 or 2. In some embodiments, -L¹-R is absent and L³ is selected from ^{#}-(CH₂-CH₃-O)ₘ-CH₂-CH₂-, ^{#}-(CH₂)ₘ-, and 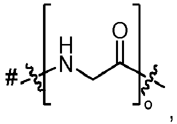 wherein ^{#} designates the attachment point to L². In more preferred embodiments, -L¹-R is absent and L³ is ^{#}-(CH₂-CH₃-O)ₘ-CH₂-CH₂-, wherein ^{#} designates the attachment point to L². The index m in ^{#}-(CH₂-CH₂-O)ₘ-CH₂-CH₂- represents an integer from 1-36, preferably from 2-24, 2-18, 2-12 or 2-6. Most preferably, m is 2.

In some embodiments, -L¹-R is absent and the linker unit -L²-L³- connecting the sugar moieties G with the chelator D has a length of 10-30 Å, preferably 14-20 Å. For instance, the length of -L²-L³- can be about 11 Å, 12 Å, 13 Å, 14 Å, 15 Å, 16 Å, 17 Å, 18 Å, 19 Å, 20 Å, 21 Å, 22 Å, 23 Å, 24 Å, 25 Å, 26 Å, 27 Å, 28 Å or 29 Å.

In a preferred embodiment, the compound is

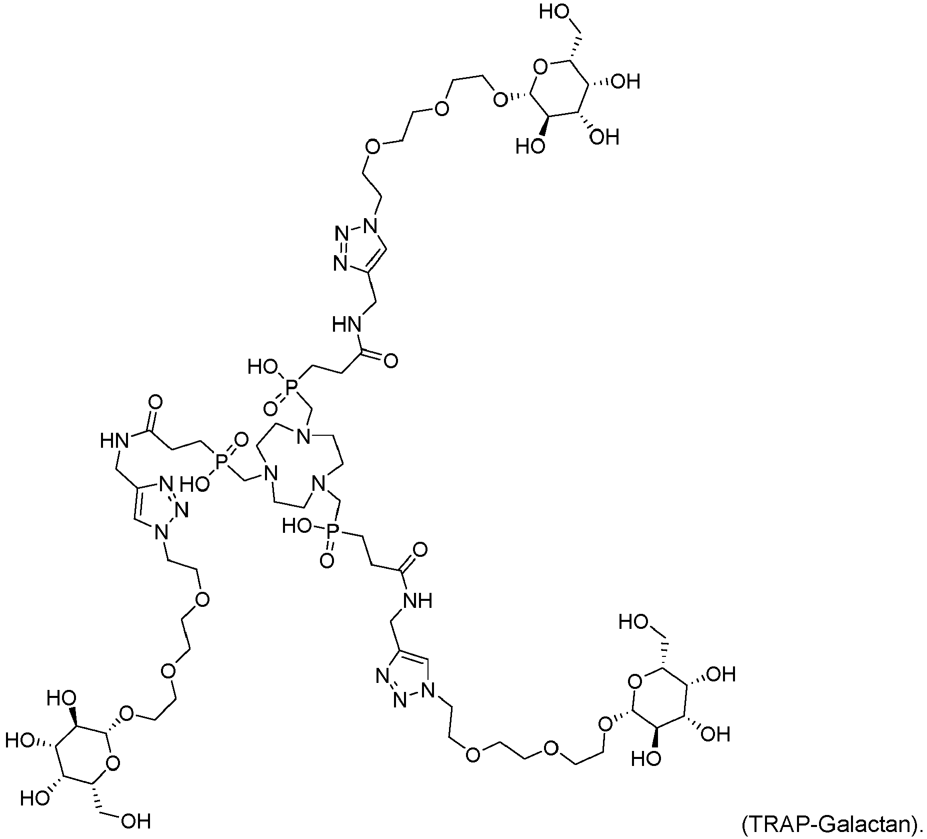

In a preferred embodiment, -L¹-R and L³ are both absent. In a more preferred embodiment, the compound is

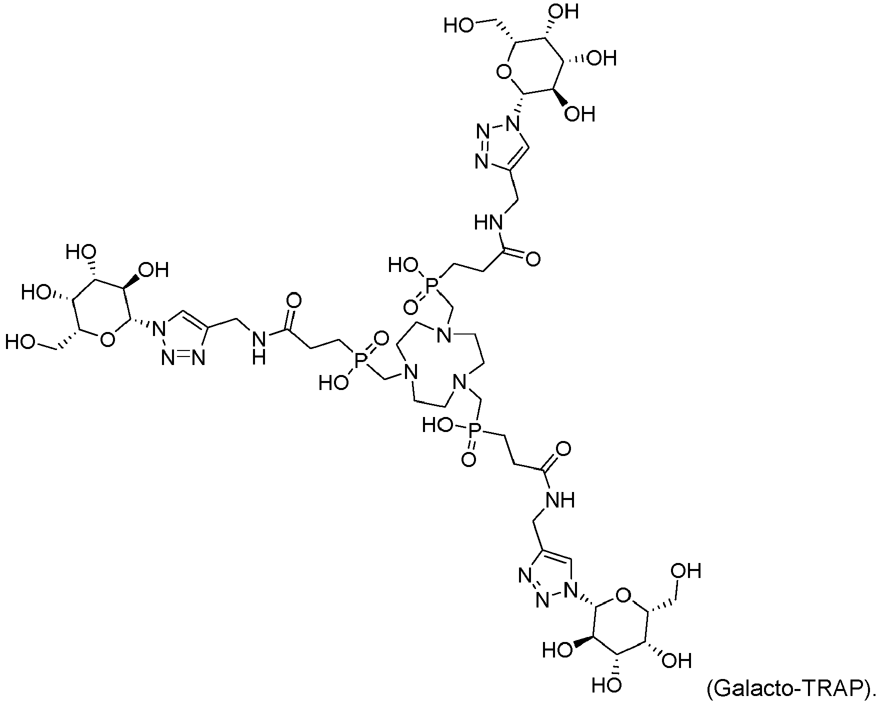

D is a chelating group (chelator), a prosthetic group or a [¹⁸F]F accepting group.

The term "chelator" or "chelating group" is known in the art and refers to organic compounds that are polydentate ligands that form two or more coordinate bonds with a radionuclide. The chelator may contain different donor groups for metal complexation such as oxygen, nitrogen, sulphur, (carboxyl, phosphonate, hydroxamate, amine, thiol, thiocarboxylate or derivatives thereof) and comprises acyclic and macrocyclic chelators such as polyaminopolycarboxylic ligands. The introduction of the radionuclide into the chelator is typically performed after conjugation of the chelator to the remainder of the molecule.

The term "prosthetic group" is known in the art and refers to a bifunctional labelling agent that is a small organic molecule that can be easily radiolabelled, for example with a radionuclide, and conjugated to a second molecule, e.g. a biomolecule such as a sugar containing molecule. Generally, prosthetic groups are conjugated to amines, thiols or carboxylic acid functions present in the second molecule. Also conjugation via click chemistry is possible.

The term "[¹⁸F]F accepting group" refers to a group that can be selectively covalently modified with [¹⁸F]F.

In some embodiments, D is a chelating group and coordinates to (chelates) a radionuclide. The radionuclide is preferably selected from [⁶⁸Ga]Ga, [¹⁸F]AlF, [⁸⁹Zr]Zr, [⁶⁴Cu]Cu and [⁸⁶Y]Y, more preferably [⁶⁸Ga]Ga or [¹⁸F]AlF, most preferably [⁶⁸Ga]Ga.

In some embodiments, the chelator is selected from the group consisting of NODAGA (1,4,7-triazacyclononane-1-glutaric acid-4,7-diacetic acid), NOTA (1,4,7-triazacyclononane-1,4,7-triacetic acid), DOTAGA (1,4,7,10-Tetraazacyclododecane-1-glutaric acid-4,7,10-triacetic acid), DO3A (1,4,7,10-Tetraazacyclododecane-1,4,7-triacetic acid), HBED (N,N'-di(2-hydroxybenzyl)-ethylenediamine-N,N'-diacetic acid), NCS-DTPA (S-2-(4-Isothiocyanatobenzyl)-diethylene-triamine pentaacetic acid), p-NCS-Bz-DFO (N1-hydroxy-N1-(5-(4-(hydroxy(5-(3-(4-isothiocyanatophenyl)thioureido)pentyl)amino)-4-oxobutanamido)pentyl)-N4-(5-(N-hydroxyacetamido)pentyl)succinamide), FusC (Fusarinine C), DafC (Diacetyl-Fusarinine C), CNAAZTA (1,4-bis(carboxymethyl)-6-[bis(carboxymethyl)]amino-6-(9-carboxynonyl)-perhydro-1,4-diazepine), NOPO (3-(((4,7-bis((hydroxy(hydroxymethyl)-phosphoryl)methyl)-1,4,7-triazonan-1-yl)methyl)(hydroxy)-phosphoryl)propanoic acid), TRAP (3,3',3"-(((1,4,7-triazonane-1,4,7-triyl)tris(methylene))tris(hydroxyphosphoryl))tripropanoic acid, DOTPI (1,4,7,10-tetraazacyclododecane-1,4,7, 10-tetrakis[methylene(2-carboxyethylphosphinic acid)), DOTAZA (1,4,7,10-Tetra-azacyclododecan-1,4,7,10-tetra-azidoethylacetic acid), sacrophagine-based chelators, and TE2A derivatives such as CB-TE2A (2,2'-(1,4,8,11-tetraazabicyclo[6.6.2]hexadecane-4,11-diyl)diacetic acid).

The radionuclide is preferably a positron-emitting isotope of a metal or halogen suitable for PET imaging. Suitable radionuclides to be coordinated to the chelating group include but are not limited to [⁸⁹Zr]Zr, [⁴⁴Sc]Sc, [⁴⁵Ti]Ti, [⁵¹Mn]Mn, [⁶⁴Cu]Cu, [⁶¹Cu]Cu, [⁶³Zn]Zn, [⁶⁸Ga]Ga, [¹¹C]C [¹²⁴I]I, and [¹⁸F]AlF. In some embodiments, the radionuclide is a metal ion such as [⁶⁸Ga]Ga, [⁸⁹Zr]Zr, [⁴⁴Sc]Sc, [⁶⁴Cu]Cu or [⁸⁶Y]Y, more preferably [⁶⁸Ga]Ga or [⁶⁴Cu]Cu. Most preferably, the radionuclide is [⁶⁸Ga]Ga. In some other embodiments, the radionuclide is a halide such as [¹²⁴I]I or [¹⁸F]AlF, most preferably [¹⁸F]AlF. In some embodiments, the radionuclide is selected from [⁶⁸Ga]Ga and [¹⁸F]AlF.

It is to be understood that various factors may be included in the selection of a suitable positron-emitting isotope. Such factors include a sufficient half-life of the isotope to allow preparation of a diagnostic composition prior to administration to the patient, and a sufficient remaining half-life to yield sufficient activity to allow non-invasive measurement by PET. The isotope should have a sufficiently short half-life to limit patient exposure to unnecessary radiation. Suitable isotopes to be coordinated by the chelating groups of the present invention are ⁶⁸Ga and [¹⁸F]AlF.

Preferred prosthetic groups of the present invention are labelled with radionuclides of halogens such as iodine, bromine or fluorine, preferably fluorine, more preferably [¹⁸F]F. The introduction of the radionuclide into the prosthetic group in typically performed before conjugation of the prosthetic group to the remainder of the molecule (e.g. conjugating D to L¹-R-(L²-L³-G)ₙ or (L²-L³-G)ₙ).

In some embodiments the prosthetic group is selected from a group comprising but not limited to 4-[¹⁸F]fluorophenacyl bromide, N-succinimidyl-4-[¹⁸F]fluorobenzoate ([¹⁸F]SFB), N-succinimidyl-4-([¹⁸F]fluoromethyl)benzoate, 4-[¹⁸F]fluorobenzaldehyde, ¹⁸F-6-fluoropyridine-3-carboxylic acid 2,3,5,6-tetrafluorophenyl ester ([¹⁸F]F-Py-TFP), silicon-containing building blocks such as N-Succinimidyl 3-(di-tert-butyl[¹⁸F]fluorosilyl)benzoate ([¹⁸F]SiFB), carbohydrate-based prosthetic groups, such as [¹⁸F]fluoro-deoxyglucose, preferably 2-[¹⁸F]fluoro-2-deoxyglucose ([¹⁸F]FDG), and [¹⁸F]fluoro-deoxymannose, preferably [¹⁸F]2-fluoro-2-deoxymannose, or derivatives thereof, maleimide-based and heterocyclic methylsulfonebased ¹⁸F-synthons, ¹⁸F-labelled prosthetic groups such as ¹⁸F-azides or ¹⁸F-alkynes permitting labelling via click chemistry, ¹⁸F-labelled organotrifluoroborates and [¹⁸F]fluoropyridines. The preferred prosthetic group is ¹⁸F-6-fluoropyridine-3-carboxylic acid 2,3,5,6-tetrafluorophenyl ester (F-Py-TFP). The linkers are designed to have a N-terminal amino function allowing labelling via any prosthetic group, which reacts with an amine.

In some embodiments a chelator-based labelling approach using aluminum-fluoride ([¹⁸F]AlF) is applied for radiofluorination.

In some embodiments, D is a [¹⁸F]F accepting group. In some embodiments, direct labelling of the compound of the present invention is achieved by attaching a [¹⁸F]F accepting group to the remainder of the molecule. For instance, N,N,N-trimethyl-5-[(2,3,5,6-tetrafluorophenoxy)carbonyl]pyridin-2-aminium trifluoromethanesulfonate can be conjugated to the amino function of R group under release of tetrafluorophenol. The trimethylammonium group acts as a leaving group and can be replaced by, e.g. [¹⁸F]F.

Also contemplated is the use of para-substituted di-tert-butylfluorosilylbenzene structural motif, which is known as the silicon-fluoride acceptor (SiFA), for incorporating radioactive [¹⁸F]F into the compound of the present invention. Labelling with [¹⁸F]F is performed by isotopic exchange of [¹⁹F]F from SiFA with [¹⁸F]F.

In some embodiments, the compound has the following structure 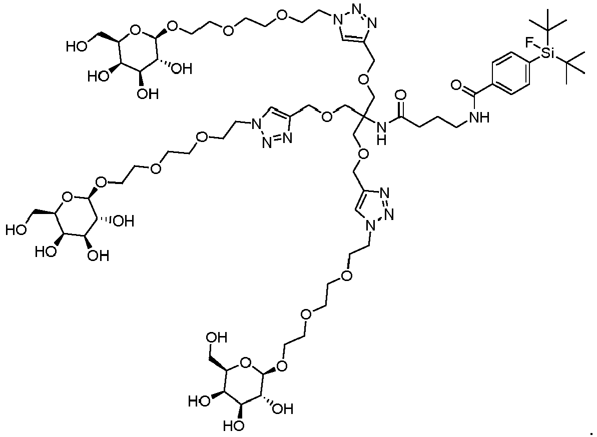 In some embodiments, the fluor of the tert-butylfluorosilylbenzene moiety is ¹⁸F.

In some embodiments, the compound has the following structure 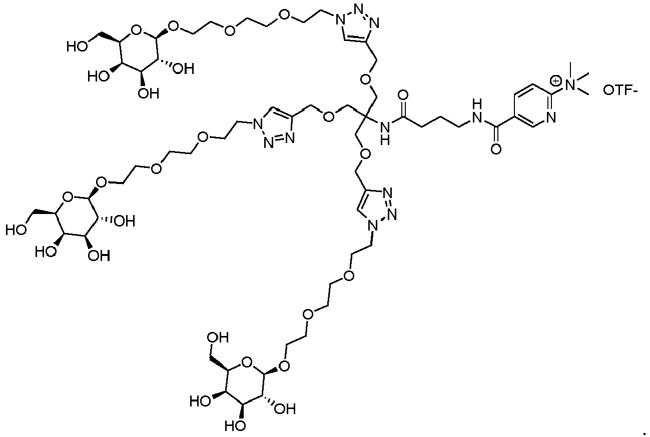 In some embodiments, the trimethylammonium group is replaced with ¹⁸F.

The sugar moiety G is galactose (Gal, D-galacto-hexopyranose), galactose with an acetylated amino residue at position 2 (GalNAc, 2-(acetylamino)-2-deoxy-D-galactose), or lactose (Lac). More preferably, G is Gal or GalNAc. The sugar moiety may be connected to the linker L³ via an O-glycosidic linkage, an N-glycosidic linkage, an S-glycosidic linkage or a C-glycosidic linkage. In a preferred embodiment, the linkage is O-glycosidic.

The present invention further relates to pharmaceutical compositions, preferably for diagnostic use, comprising the compound described herein and a pharmaceutically acceptable additive.

The present invention also relates to a method of imaging cells as described herein. The method of imaging cells described herein makes use of the compound described herein.

The present method of imaging cells makes us of established methods of imaging. Preferably, positron emission tomography (PET) is used for imaging the cells. PET is a nuclear imaging methology that detects pairs of gamma rays emitted indirectly by a positron-producing radionuclide. Compared to other radioimaging methologies such as Single Photon Emission Computed Tomography (SPECT), PET is known to show higher sensitivity, better spatial resolution, greater signal to noise ratio, and superior tracer quantification in both preclinical and clinical applications.

Combining the functional imaging obtained by PET, which depicts the spatial distribution of metabolic or biochemical activity in the body, with computer tomography (CT) allows to more precisely align or correlate the results with the anatomy of the body. Combination of PET with magnetic resonance imaging (MRI) combines the exquisite structural and functional characterization of tissue provided by MRI with the extreme sensitivity of PET imaging of metabolism and tracking of uniquely labeled cell types or cell receptors.

Radiopharmaceuticals such as the compounds of the present invention are of great interest for patient management in a diversity of clinical settings comprising liver surgery and liver transplantation, diagnosis and treatment monitoring of cancer, as well as other liver diseases like alcoholic liver cirrhosis and liver fibrosis. The optimal liver function assessment will allow a precise evaluation of the different liver segments aimed at better predicting liver failure following major liver surgery.

The compounds of the present invention overcome the drawbacks of the human GSA-based radiotracers, e.g. ^{99m}Tc-GSA, in the art because they can be easily prepared and put to practice in clinical routine. Surprisingly, the present inventors have found that the compounds of the present invention show higher affinity for the target receptors compared to the GSA-based radiotracer. Without being bound by theory, it is thought that due to the lower molecular mass, elimination from the body is faster resulting, at least for later time points, in improved liver to organ ratios compared with GSA-based radiotracer such as ^{99m}Tc-GSA.

In one aspect, the present invention provides a method of imaging hepatocytes, comprising a) contacting the hepatocyte with a compound of the present invention and b) visualizing the compound that is in contact with the hepatocyte.

In a further aspect, the present invention provides a method of determining functional hepatic reserve, comprising a) contacting the liver with the compound of the present invention, and b) visualizing the compound that is in contact with the liver.

In some embodiments, the visualizing is performed via positron emitting computer tomography (PET).

The methods of the invention may be performed *in vivo* or *in vitro.*

The contacting of the hepatocyte or the contacting of the liver with the compound can be achieved, for example, by administering the compound to a patient. Administration to the patient can be by injection, in particular by intravenous injection.

For administration to a patient, the compound is formulated in a suitable dosage form. One suitable dosage form is a pharmaceutical or diagnostic composition, comprising at least on additive, such as saline.

In some embodiments, the molar activity of the radiolabelled compound ranges from about 50 MBq/nmol up to about 33 GBq/nmol.

In some embodiments, if D is a chelator group and the radionuclide is [⁶⁸Ga]Ga, the molar activity of the radiolabelled compound is about 50-500 MBq/nmol. In preferred embodiments, the molar activity of the radiolabelled compound is about 50-450 MBq/nmol, about 50-400 MBq/nmol, about 50-450 MBq/nmol, about 50-400 MBq/nmol, about 100-500 MBq/nmol, about 100-450 MBq/nmol, about 100-400 MBq/nmol, or about 120-400 MBq/nmol. In a more preferred embodiment, the molar activity of the radiolabelled compound is about 100-400 MBq/nmol.

In some embodiments, if D is a prosthetic group and the radionuclide is [¹⁸F]F or if D is an [¹⁸F]F acceptor group, the molar activity of the radiolabelled compound ranges from 1000 MBq/nmol up to 33 GBq/nmol. In preferred embodiments, the molar activity of the radiolabelled compound is about 3000 MBq/nmol - 20 GBq/nmol, about 3500 MBq/nmol - 10 GBq/nmol, about 3500-5000 MBq/nmol, or about 3500-4500 MBq/nmol. In a more preferred embodiment, the molar activity of the radiolabelled compound is about 3500-5000 MBq/nmol.

In some embodiments, if D is a chelator group and the radionuclide is [¹⁸F]AlF, the molar activity of the radiolabelled compound ranges from 10 MBq/nmol up to 2 GBq/nmol.

In some embodiments, if the radiolabelled compound is produced via isotopic exchange reaction, the molar activity is about 50-500 MBq/nmol, more preferably about 50-450 MBq/nmol.

### Examples

The compounds of the present invention can be prepared by a combination of methods known in the art including the procedures described in schemes 1, 2 and 3 below. The following reaction schemes are meant only to exemplify and do not limit the invention.

The labelling precursor NODAGA-TriGalactan was obtained in a seven step synthesis (Scheme 1). For the first reaction step, Boc-protected Tris **(1)** was reacted with propargylbromide to give the respective ether conjugate **(2).** Next, Cu-catalyzed click chemistry was used to attach three galactose moieties to the backbone followed by removal of the Bocprotection group using trifluoroacetic acid **(3).** In a subsequent step γ-amino butyric acid was introduced as a linker on the free amino function of Tris **(4).** Coupling of *R*-NODAGA and subsequent removal of all acetyl protection groups from the galactoses yielded NODAGA-TriGalactan **(6).**

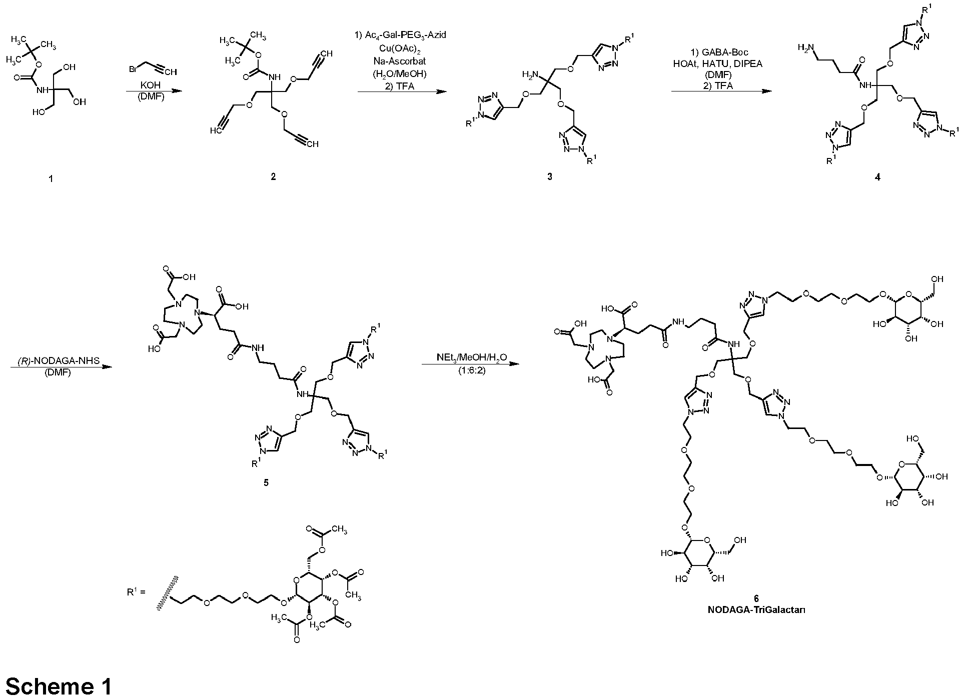

The labelling precursor TRAP-Galactan (4) was obtained in a four step synthesis (Scheme 2). For the first reaction step the chelator TRAP-Pr (1) was functionalized with propargylamine to give TRAP-Alkyne₃ **(2).** Next, Cu-catalyzed click chemistry was used to attach three galactose moieties to the chelator followed by demetallation of the resulting Cu-complex. **(3).** Subsequent removal of all acetyl protection groups from the galactoses yielded TRAP-Galactan **(4).**

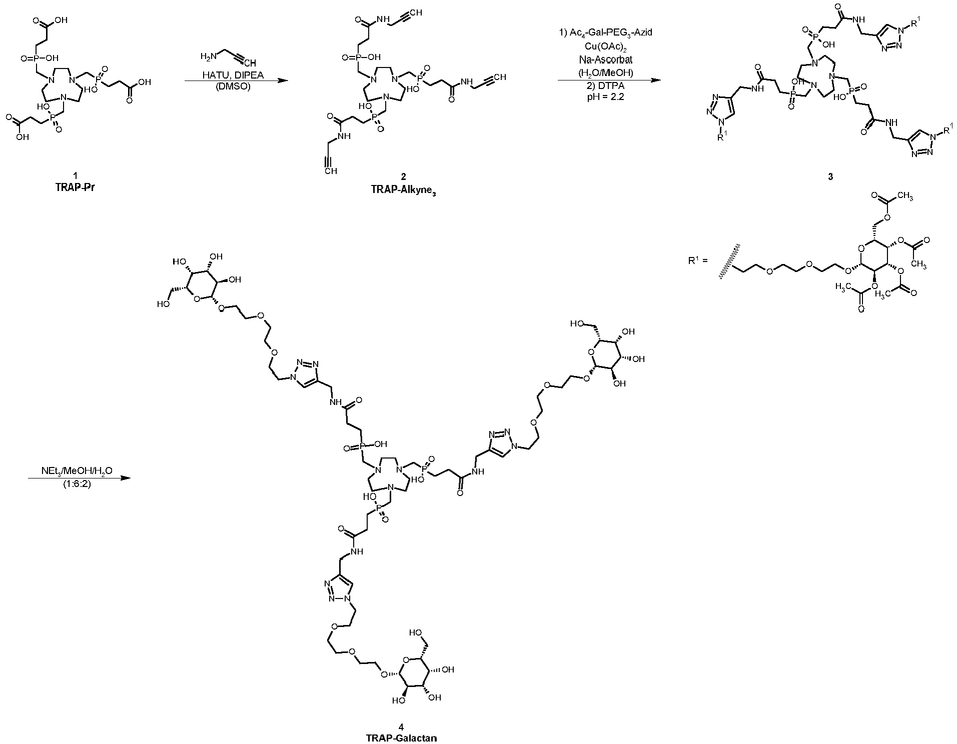

The labelling precursor Galacto-TRAP **(6)** was obtained in a four step synthesis (Scheme 3). For the first reaction step the chelator TRAP-Pr **(1)** was functionalized with propargylamine to give TRAP-Alkyne₃ **(2).** Next, Cu-catalyzed click chemistry was used to attach three galactose moieties to the chelator followed by demetallation of the resulting Cu-complex. **(5).** Subsequent removal of all acetyl protection groups from the galactoses yielded Galacto-TRAP **(6).**

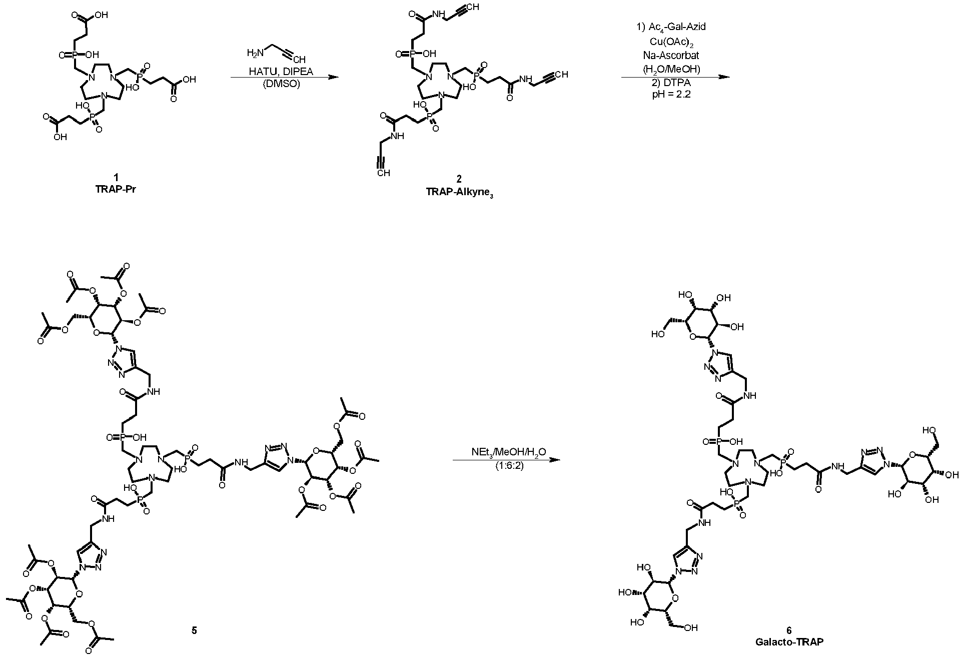

### Example 1: Synthesis of NODAGA-TriGalactan

### Step 1: Synthesis of Boc-Tris(Propargyl)₃ (2)

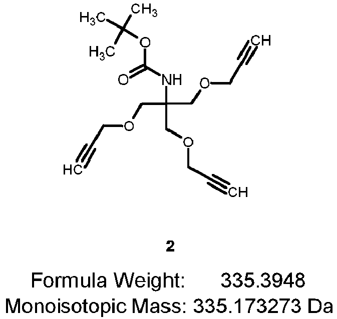

To a 100 mL round bottom flask 500 mg (2.3 mmol, 1 eq) of Boc-protected Tris **((1)** in Scheme 1) were added as a solid and dissolved in 6 mL of dry DMF. The solution was cooled on ice followed by addition of 1.5 mL of propargylbromid in toluene (80 %, 13.9 mmol, 6.2 eq) as well as 1 g (17.9 mmol, 7.9 eq) of fine powdered KOH. The reaction mixture was stirred on ice for 15 min and was allowed to warm up to room temperature afterwards. For workup, 20 mL of ethylacetate were added and the resulting solution was transferred into a separation funnel where the organic layer was washed three times with 20 mL of distilled water. The organic layer was separated and the aqueous extracts were washed twice with 20 mL of ethylacetate.

The combined organic fractions were dried over NaSO₄, filtered and all volatiles removed *in vacuo* yielding the raw product as a yellowish oil which was subsequently purified on column chromatography using a gradient of n-hexane/ethylacetate (90/10 - 40/60). The product containing fractions were pooled and all volatiles were removed *in vacuo* to yield the product as a yellowish oil that solidified upon storage in the refrigerator.

**¹H-NMR** (CDCl₃): δ (ppm) = 4.91 (s, 1 H, NH), 4.13 (m, 6H, CH₂-C≡C), 3.75 (s, 6 H, O-CH₂), 2.48 (t, 3 H, ⁴*J* = 1.8 Hz, C≡CH), 1.41 (s, 9H, CH₃).

**MALDI-MS** (m/z) = 336.3 [M+H]+, 358.3 [M+Na]⁺.

### Step 2: Synthesis of Tris(Ac₄-Gal)₃ (3)

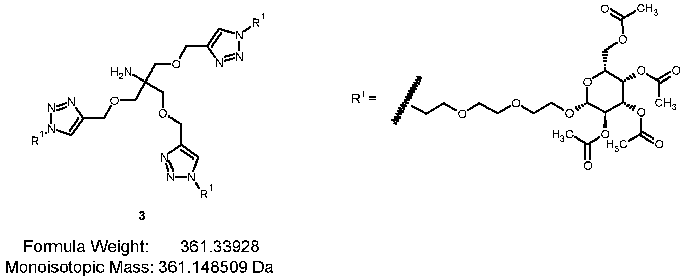

Compound **2** (24.9 , 74.2 µmol, 1 eq) was weighed into a 15 mL Falcon-Tube and a solution of 135 mg (267 µmol, 3.6 eq) 1-Azido-1-deoxy-b-D-Galactopyranoside in 600 µL of MeOH was added. The reaction was started by subsequent addition of an aqueous solution of Cu(OAc)₂*H₂O (17.8 mg, 89.1 µmol, 1.2 eq) and sodium ascorbate (586 mg, 3 mmol, 40 eq) and the resulting mixture was heated on a waterbath at 60 °C for 1 h. For workup all volatiles were removed *in vacuo* and for Boc-deprotection the residue was resuspended in 4 mL of TFA/TIPS/H₂O (95/2.5/2.5) and incubated for 1 h at room temperature. After removal of TFA under a stream of Argon, the raw product was dissolved in 1.6 mL of H₂O/30 % MeCN (vol/vol) and substracted to semipreparative RP-HPLC (30-50 % B in 20 min). The product was obtained as a colorless oil (27 mg 14.5 µmol, 20 %).

**Analytical HPLC** (ReproSil Pur, 30-100 % B in 15 min, 1.0 mL/min) t_{R} = 8.5 min (70 % B)

**MALDI-MS** (m/z) = 1750.2 [M+H]+, 1772.2 [M+Na]⁺.

### Step 3: Synthesis of GABA-Tris(Ac₄-Gal)₃ (4)

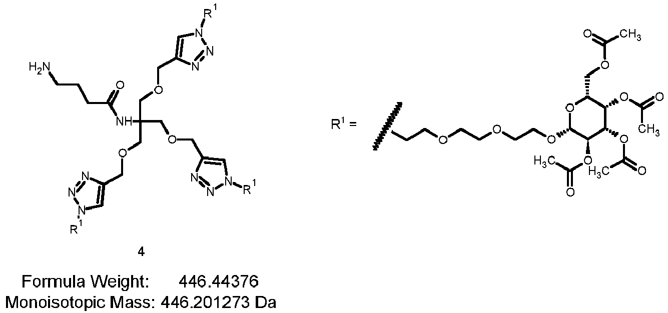

In a V-shaped vial HOAt (3.95 mg, 29.0 µmol, 2 eq), HATU (11.0 mg, 29.0 µmol, 2 eq), and Boc-GABA (5.89 mg, 29.0 µmol, 2 eq) were dissolved in 100 µL of anhydrous DMF and DIPEA (15.2 µL, 87.0 µmol, 6 eq). After 10 minutes of preactivation this solution was added dropwise to a solution of compound **3** (27.0 mg, 14.5 µmol, 1 eq) in 350 µL of dry DMF and 15.2 µL (87.0 µmol, 6 eq) DIPEA. The reaction mixture was allowed to stand at room temperature for 2 h before 500 µL of water were added to deactivate the remaining coupling agents. The solution was transferred to a 10 mL round bottom flask and all volatiles were removed *in vacuo.* For Boc-deprotection the residue was resupended in 1 mL TFA/TIPS/H₂O (95/2.5/2.5) and incubated for 1 h at room temperature. After removal of TFA under a stream of Argon the raw product was dissolved in 660 µL H₂O/30 % MeCN and substracted to semipreparative RP-HPLC (30-45 % B in 20 min). The product was obtained as as colorless oil (15.9 mg, 8.16 µmol, 56%).

**Analytical HPLC** (ReproSil Pur, 20-80 % B in 15 min, 1.0 mL/min) t_{R} = 11.2 min (65 % B).

**MALDI-MS** (m/z) = 1835.8 [M+H]⁺, 1857.2 [M+Na]⁺.

### Step 4: Synthesis of NODAGA-TriGalactan (6)

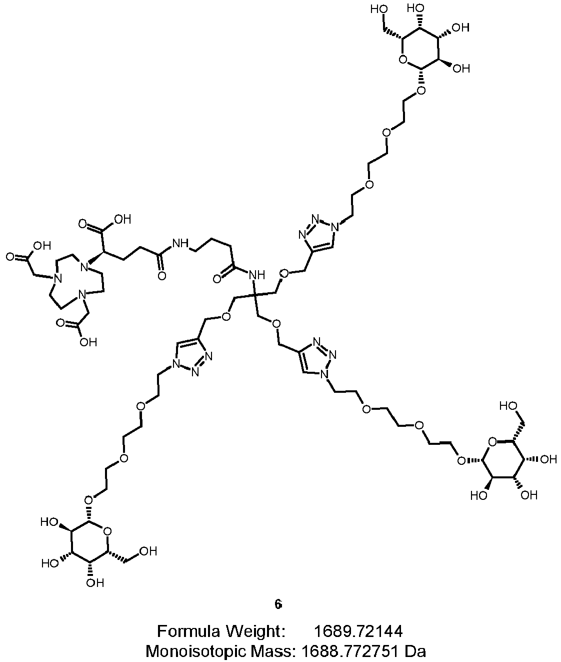

(*R*)-NODAGA-NHS (7.17 mg, 9.79 µmol, 1.2 eq) was weighed into a 1.5 mL eppendorf reaction vial and was dissolved in 100 µL of anhydrous DMF and DIPEA (4.27 µL, 24.5 µmol, 3 eq). This mixture was then added dropwise to a solution of compound **4** (15.9 mg, 8.16 µmol, 1 eq) in 300 µL of anhydrous DMF and DIPEA (8.53 µL, 49.0 µmol, 6 eq). The pH was adjusted by additition of another 3 eq DIPEA and the mixture was stirred over night at room temperature. For removal of the acetyl protection groups the reactions mixture was reduced to dryness and the residue was dissolved in 3 mL of NEt₃/MeOH/H₂O (1/6/2). After 12 hours all volatiles were removed *in vacuo* and the raw product was substracted to purification via semi-preparative RP-HPLC (12 % B isocratic). After lyophylisation the product was obtained as a white solid (4.48 mg, 2.34 µmol, 29 %).

**Analytical HPLC** (ReproSil Pur, 5-60 % B in 15 min, 1.0 mL/min) t_{R} = 7.2 min (32 % B).

**MALDI-MS** (m/z) = 1689.7 [M+H]+.

### Example 2: Labelling of NODAGA-TriGalactan with ⁶⁸Ga- and ^{nat}Ga-gallium

### ⁶⁸Ga-NODAGA-TriGalactan

For labeling with Gallium-68 a fractionated elution approach was applied. An overall activity of 200 MBq was eluted from the ⁶⁸Ga/⁶⁸Ge generator in 1.5 mL fractions and the activity content of each fraction was calculated using a dose calibrator. From the fraction containing the highest activity, 550 µL were added to a mixture of 5 nmol (1 mM, 5 µL) of the labeling precursor in 100 µL of a 1 M NaOAc/HOAc-buffer (pH = 5). The mixture was heated to 56 °C for 10 min and completion of the labelling reaction was monitored using radio-TLC and radio-HPLC (ReproSil Pur, Gradient: 10-60 %B in 15 min). If required the labelled compound was further purified via fixation on a Waters (Milford, Massachusetts, USA) Sep Pak Ct 18 Light cartridge. For preconditioning the cartridge was flushed with 5 mL of EtOH followed by 10 mL of water and 1mL of air. The labelling solution was loaded onto the cartridge and 10 mL of water were used for washing. The final compound was eluted using 300 µL of EtOH/H₂O (1:1) and diluted with PBS for further usage.

### ^{nat}Ga-NODAGA-TriGalactan

Complexation with ^{nat}Ga was done accordingly to a published procedure (A. Wurzer et al. J Nucl Med, 2020). A 2 mM stock solution of NODAGA-TriGalactan (500 µL, 1 µmol, 1 eq) was mixed with 150 µL of a 20 mM ^{nat}GaBr₃ solution (3 µmol, 3 eq) and incubated for 10 min at 56 °C.

*RP-HPLC* (ReproSil Pur, 5-60 % B in 15 min) t_{R} = 7.2 min. *Calculated monoisotopic mass* (C₆₈H₁₁₃GaN₁₄O₃₅): 1754.7 Da; found (m/z) = 1756.1 [M+H]⁺, 1778.0 [M+Na]⁺, 1794.0 [M+K]⁺.

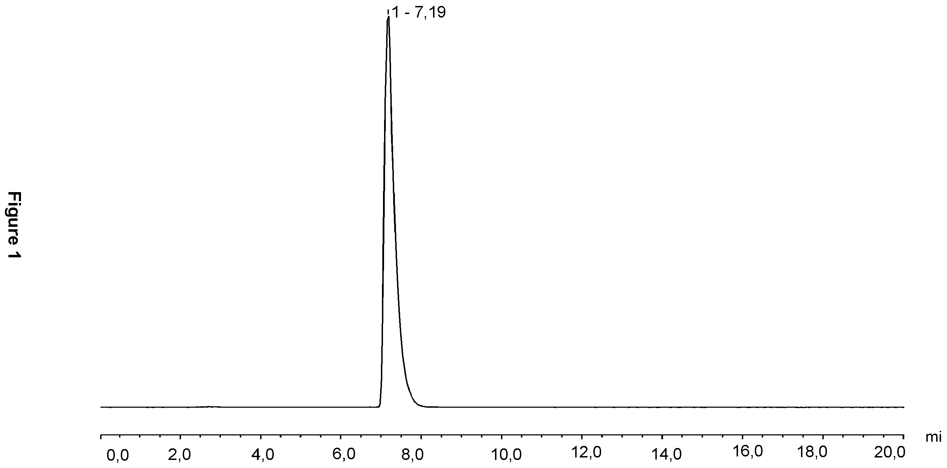

Radiolabelling of NODAGA-TriGalactan with ⁶⁸Ga was achieved in high radiochemal yields and purity as determined by radio-HPLC (> 99 %) (Fig. 1) and radio-TLC (> 98 %). Molar activities were usually between 8 - 10 MBq/nmol. Labelling with ^{nat}Ga with a 3-fold molar excess over the labeling precursor occurred instantly and led to formation of [^{nat}Ga]Ga-NODAGA-TriGalactan in quantitative yields and high purity (> 97 %) as determined by HPLC and MS analysis.

### Example 3: Synthesis of TRAP-Galactan

### Step 1: Synthesis of TRAP-Alkyne₃

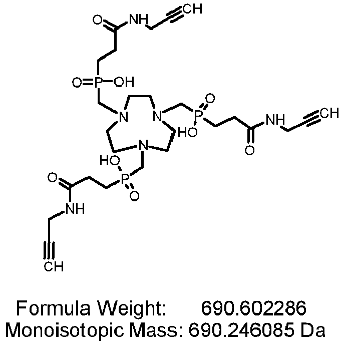

TRAP-Pr (200 mg, 346 µmol, 1.00 eq) was weighed into a 10 mL round bottom flask and got dissolved in 1 mL DMSO and 724 µL DIPEA (4.16 mmol, 12.0 eq). The solution stirred for about 5 min at room temperature before propargylamine hydrochloride (158 mg, 1.73 mmol, 5.00 eq) and HATU (1.18 g, 3.12 mmol, 9.00 eq) were added as a solid. The mixture stirred for 1 h at room temperature, then all volatiles were removed *in vacuo* and the oily residue got transferred into a 15 mL Falcon tube followed by addition of 3 x 500 µL Millipore water to deactivate the remaining coupling agents. After centrifugation (10 min, 3000 RPM) the supernatant was syringe filtered, acidified by addition of 25 µL of TFA and substracted to semipreparative RP-HPLC purification (12 % B isocratic). After lyophylization the product was obtained as a colorless oil (232 mg, 288 µmol, 83 %).

**RP-HPLC** (5-15 % B in 15 min) t_{R} = 11.7 min.

**ESI-MS** (m/z) = 691.2 [M+H]+.

### Step 2: Synthesis of TRAP-Galactan

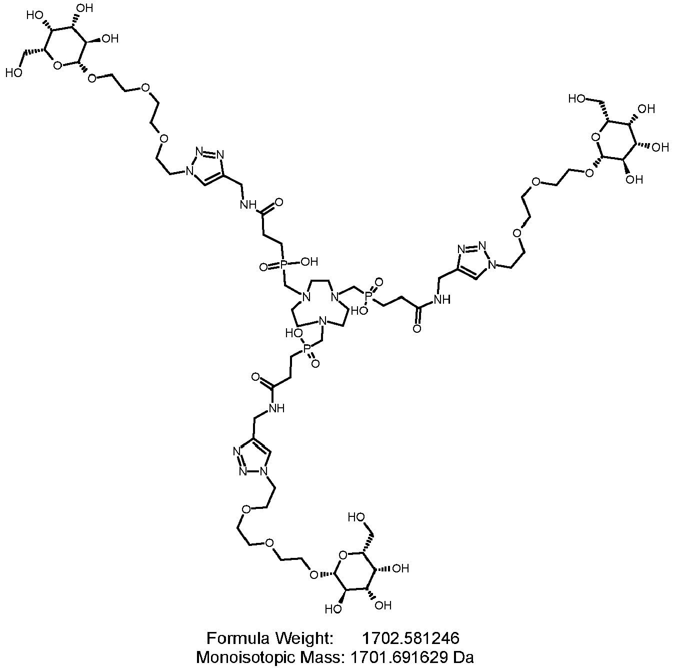

TRAP-Alkyne₃ (17.7 mg, 22.0 µmol, 1.00 eq) was dissolved in 35 µL Millipore Water and was added to a solution of 40.0 mg (79.1 µmol, 3.60 eq) Ac₄-Gal-PEG₃-Azid in 200 µL of MeOH. Furthermore aqueous solutions of 5.27 mg (26.4 µmol, 1.20 eq) Cu(OAc)*H₂O and 174 mg (880 µmol, 40.0 eq) of sodium ascorbate were added and the resulting mixture was stirred for 1 h at 60 °C. After that the reaction mixture was centrifuged (14 000 rpm, 5 min) and the supernatant was added to a solution of 330 mg (662 µmol, 30.0 eq) DTPA in 300 µL of millipore water. The pH was adjusted to 2.2 using concentrated HCI and the resulting solution was heated to 60 °C for 1 h. Completion of the demetallation reaction was monitored by MS and the acetyl protected intermediate was isolated by semi-preparative RP-HPLC (38 % B isocratic). Fractions of the intermediate were collected and reduced to dryness before 3 mL of NEt₃/MeOH/H₂O (1/6/3) were added for deacetylation. After 24 h at room temperature all volatiles were removed *in vacuo* and the residue was dissolved in 1.5 mL of H₂O/10 % MeCN. Purification via semi-preparative RP-HPLC (5-10 % B in 25 min) followed by lyophylization yielded 21.8 mg (11.3 µmol, 51 %) of a waxy solid.

**RP-HPLC** (5-15 % B in 15 min) t_{R} = 13.5 min.

**MALDI-MS** (m/z) = 1748.3 [M+2Na]⁺, 1762.3 [M+Na+K]⁺.

### Example 4: Synthesis of Galacto-TRAP

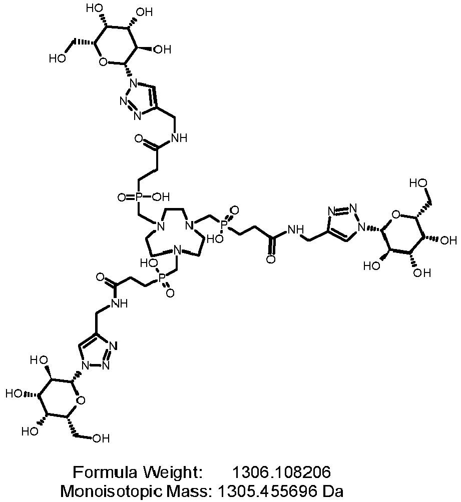

TRAP-Alkyne₃ (10.0 mg, 12.4 µmol, 1.00 eq) were dissolved in 20 µL of Millipore water and was added to a solution of 1-Azido-1-deoxy-β-D-Galactose (16.7 mg, 44.6 µmol, 3.60 eq) in 150 µL hot MeOH. Furthermore aqueous solutions of 2.95 mg (14.8 µmol, 1.20 eq) Cu(OAc)*H₂O and 73.7 mg (372 µmol, 30.0 eq) of sodium ascorbate were added and the resulting mixture was stirred for 1 h at 60 °C. After that the reaction mixture was centrifuged (14 000 rpm, 5 min) and the supernatant was added to a solution of 185 mg (372 µmol, 30.0 eq) DTPA in 500 µL of millipore water. The pH was adjusted to 2.2 using concentrated HCI and the resulting solution was heated to 60 °C for 1 h. Completion of the demetallation reaction was monitored by MS and the acetyl protected intermediate was isolated by semipreparative RP-HPLC (38 % B isocratic). Fractions of the intermediate were collected and reduced to dryness before 3 mL of NEt₃/MeOH/H₂O (1/6/3) were added for deacetylation. After 24 h at room temperature all volatiles were removed *in vacuo* and the residue was dissolved in 300 µL of H₂O/5 % MeCN. Purification via semi-preparative RP-HPLC (1-10 % B in 30 min) followed by lyophylization yielded 4.83 mg (3.15 µmol, 25.4 %) of a white solid.

**RP-HPLC** (1-10 % B in 15 min) t_{R} = 8.9 min.

**MALDI-MS** (m/z) = 926.2 [2(M+2Na+K)]³⁺, 1305.8 [M+H]+, 1421.5 [M+H+3K]+.

### Example 5: Labelling of TRAP-Galactan and Galacto-TRAP with ⁶⁸Ga- and ^{nat}Ga-gallium

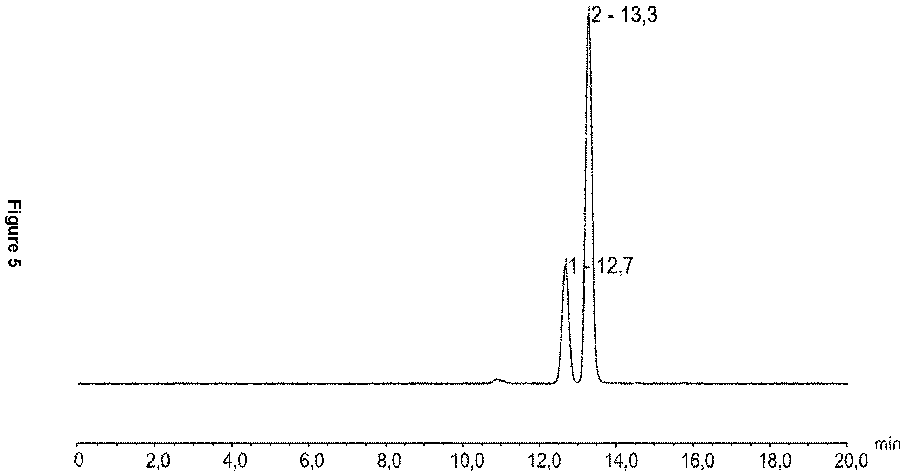

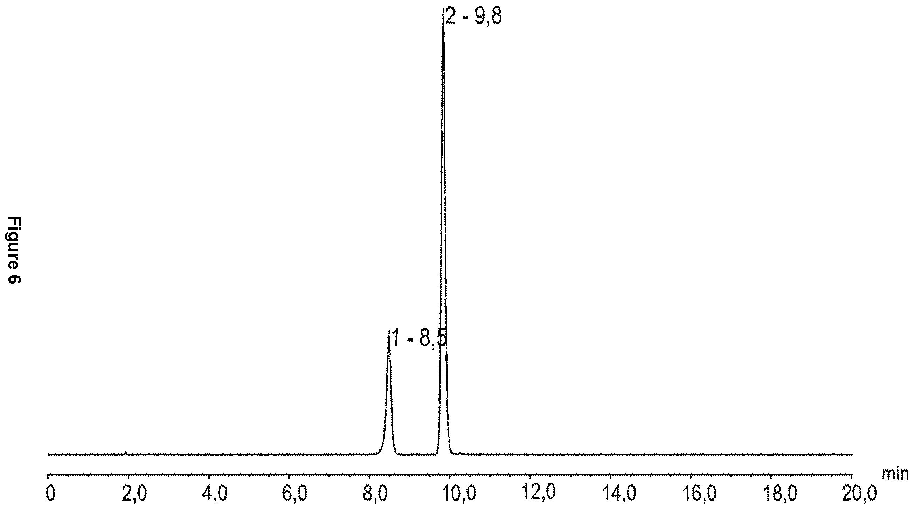

Radiolabelling of TRAP-Galactan & Galacto-TRAP with ⁶⁸Ga was achieved in high radiochemal yields and purity as determined by radio-HPLC (> 98 %) and radio-TLC (> 98 %) (Fig. 5 and 6). Molar activities were usually between 10 - 15 MBq/nmol. Labelling with ^{nat}Ga with a 1.5-fold molar excess over the labeling precursor occurred instantly and led to formation of the respective ^{nat}Ga-TRAP complexes in quantitative yields and high purity (> 97 %) as determined by HPLC and MS analysis. MS analysis also shows that both peaks correspond to the same mass and thus, might be different conformers of the desired porducts. Of interest is also that the ratio of both peaks are similar for both compounds.

### Example 6: Synthesis of reference compound ⁹⁹Tc-GSA

Preparation of the clinically established reference ligand ^{99m}Tc-GSA was done according reference (Kudo M et al. Methods Enzymol, 1994). In brief, 130 µg of GSA-Kit formulation were dissolved in 110 µL of Millipore water and 100 µL of ^{99m}Tc-Pertechnetate solution (approx. 200 MBq) were added as well as 88 nmol (22 mM, 4 µL) of a freshly prepared SnCl₂ solution in 0.1 M HCI. The mixture was gently shaken (300 1/min) and incubated for 10 min at 37 °C. Completion of the labelling reaction was monitored with Radio-HPLC (Jupiter, Gradient: 10-60 % B in 15 min). The labelled compound was then diluted with PBS without further purification. For *in vivo* studies the ^{99m}Tc-pertechnetate solution was diluted with 0.9 % saline and an activity of approx. 15 MBq/100 µL was added to reach the same molar activity as for the gallium labelled tracer.

**Radio-HPLC** (Jupiter, 10-60 % B in 15 min, 1.0 mL/min) t_{R} = 9.0 min (40 % B).

### Example 7: In vitro evaluation

### Partition coefficient and protein binding

For determination of logarithmic partition coefficients (*logD*) *n*-Octanol (500 µL) and PBS (500 µL) were pipetted into eight 1.5 mL Protein Low-Bind Eppendorf tubes followed by addition of 300 kBq of labelled compound. The vials were vortexed for 5 min at 1,500 1/min and then centrifuged for 5 min at 20,000 rcf (Eppendorf Centrifuge 5424). Aliquots of 100 µL were taken out of each phase and the amount of activity was quantified in a γ-counter (Perkin Elmer 2480 Wizard2 3"; Waltham, Massachusetts, USA).

Protein binding was determinded for different time points (2 min, 30 min, 60 min, 120 min) in triplicates using Sephadex MicroSpin G-50 columns (GE-Healthcare, Chicago, Illinois, USA). The columns were spun initially at 20,000 rcf for 1 min to remove the storage buffer. Then approx. 5 MBq of radioligand was added to 1 mL of fresh human serum and incubated at 37 °C. At each given timepoint 25 µL of the mixture was loaded on to the column, which was then spun at 20,000 rcf for 1 min. The activity content on the column (free radioligand) and in the filtrate (protein bound fraction) was quantified in a γ-counter.

### Stability studies

Ligand stability in human blood serum (n = 2) and PBS (n = 1) was determined for different time points (2 min, 30 min, 60 min, 120 min). Therefore, approx. 5 MBq of radioligand was added to 1 mL of fresh human serum or PBS and incubated at 37 °C. At each given time point 100 µL of the mixture were transferred to a 1.5 mL Eppendorf tube containing 100 µL of acetonitrile. The vial was vortexed and centrifuged for 2 min at 14,000 1/min before 100 µL of the supernatant were diluted with 200 µL of water and injected into analytical radio-HPLC. The PBS solution was directly injected into the radio-HPLC. Ligand stability was determined by integrating the areas of the signals in the radio chromatogram in relation to the signal of the intact ligand.

### In vitro binding studies

Inhibitory constant (IC₅₀) values were determined in triplicates using an isolated receptorbased assay as described in literature (Eggink LL et al. J Immunother Cancer 2018;6:28). Recombinant human ASGR1 was seeded out into Ni-coated 96 well plates (Pierce, Thermo Fisher) in PBS at a concentration of 2 µg/mL for at least 2 h at room temperature under mild agitation (200 1/min). Unbound receptor was removed by washing the wells twice with binding buffer (25 mM Tris x HCI, 150 mM NaCl, 0.05 % TWEEN 20, pH = 7.4). Next 140 µL of binding buffer as well as 20 µL of a 19 mM CaCl₂-solution were added to the wells followed by addition of either 20 µL of PBS (control) or 20 µL of [^{nat}Ga]Ga-NODAGA-TriGalactan in PBS. Final ligand concentrations were ranging from 10⁻⁵ - 10⁻¹¹ M. Lastly 20 µL of ¹²⁵I-Asialoorosomucoid (50 nM, 2.4 kBq per well) were added and the plate was incubated for 1 hour at room temperature under mild agitation (200 1/min). Supernatants were removed and the wells washed twice with 200 µL of PBS before receptor bound activity was released by addition of 200 µL of hot (60 °C) 1 M NaOH. Wells were incubated with NaOH for 10 min. Lysates were removed and the wells washed again twice with 200 µL of hot NaOH. The activity content in the lysates was quantified in a γ-counter and the values plotted in excel. Fitting of the sigmoidal binding curve and the calculation of the IC₅₀ value was done with excel's solver plugin.

[⁶⁸Ga]Ga-NODAGA-TriGalactan showed high stability in PBS (> 99 % intact tracer) and human blood serum (> 93 %) over the time course of 2 hours as determined by radio-HPLC.

Furthermore, low protein binding could be observed, and rise from 7.6 ±3.9 % (n = 3) after 2 min to 13.6 ± 3.2 % (n=3) after 120 min incubation (Table 1). Determination of *logD*-values for [⁶⁸Ga[Ga-NODAGA-TriGalactan (- 4.33 ± 0.09, n = 6) and the reference ligand ^{99m}Tc-GSA (- 1.88 ± 0.02, n = 8) revealed high hydrophilicity of the tracer.

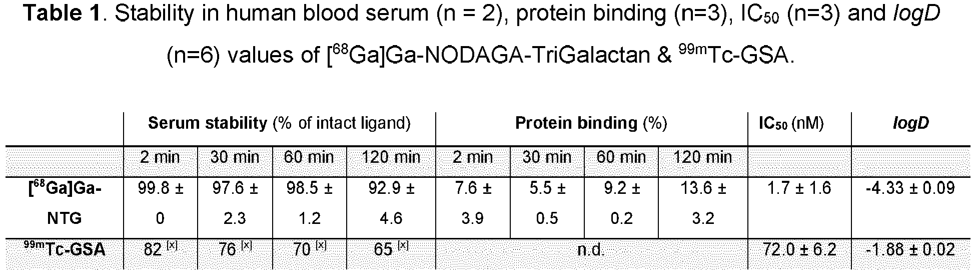

[⁶⁸Ga]Ga-TRAP-Galactan showed high stability in PBS (> 99 % intact tracer) and human blood serum (> 98 %) over the time course of 2 hours as determined by radio-HPLC. Furthermore, low protein binding could be observed, and rise from 8.2 ± 3.4 % (n = 3) after 2 min to 11.6 ± 2.2 % (n=3) after 120 min incubation (Table 2). Determination of *logD*-values for [⁶⁸Ga]Ga-TRAP-Galactan (- 4.26 ± 0.08, n = 6) and [⁶⁸Ga]Ga-Galacto-TRAP (- 4.16 ± 0.09, n = 6) revealed high hydrophilicity of both tracers.

**Table 2. Stability in human blood serum (n = 2) and proteinbinding (n=3) of [⁶⁸Ga]Ga-TRAP-Galactan.**

| **Serum stability** (% of intact ligand) | | | | **Protein binding** (%) | | | |
|---|---|---|---|---|---|---|---|
| 2 min | 30 min | 60 min | 120 min | 2 min | 30 min | 60 min | 120 min |
| 97.7 ± 0.3 | 97.6 ± 0.2 | 97.6 ± 0.1 | 97.9 ± 0.1 | 8.2 ± 3.4 | 6.4 ± 1.8 | 12.0 ± 3.1 | 11.6 ± 2.2 |

### Example 8: In vivo evaluation

### Biodistribution studies

All animal experiments were conducted in accordance with Austrian animal experiments law (BGBI. I Nr. 114/2012) and according to the institution's animal welfare standards as approved by the government of Austria (2022-0.311.708).

For biodistribution studies 6 week old female BALB/c mice (n=3) were injected with 0.1 nmol (100 µL, approx. 1 MBq) of labelled compound via the lateral tail vein and sacrificed after 10, 30, or 60 min by cervical dislocation. The mice were dissected, blood and organs (spleen, pancreas, stomach, intestine, liver, kidney, heart, lung, muscle and bone) were weighed and their activity measured in a γ-counter. For blocking experiments, mice were co-injected with 100 µL of a 277 mM Galactose/PBS solution.

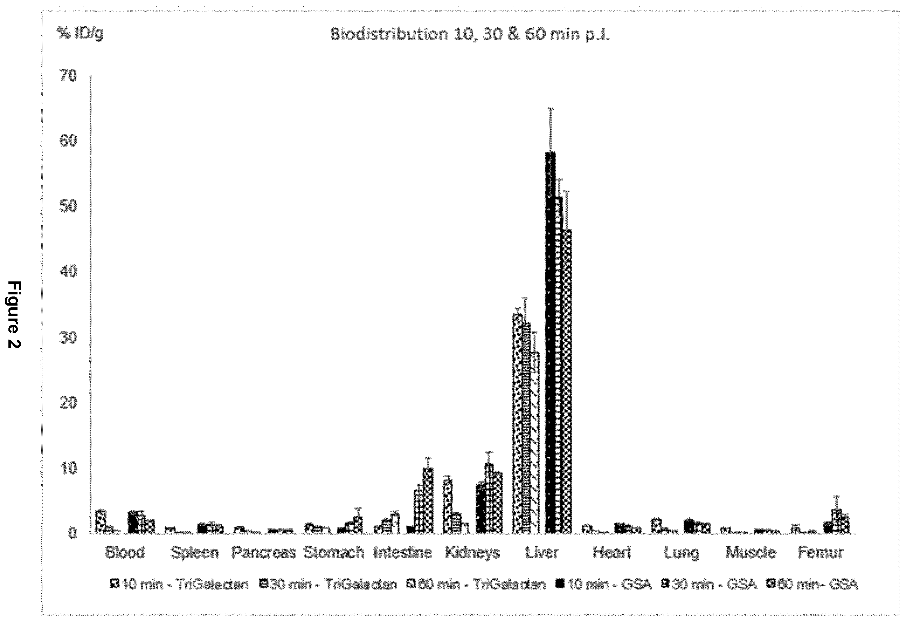

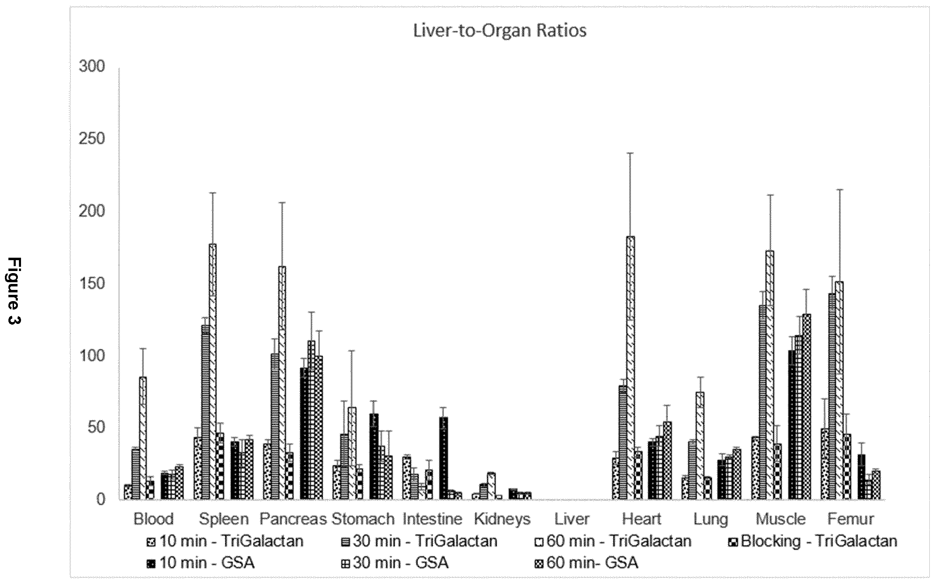

Biodistribution studies for [⁶⁸Ga]Ga-NODAGA-TriGalactan showed high liver uptake reaching its maximum already at 10 min p.i. (33.4 ± 0.9 % ID/g) and staying at a constant high level up to 60 min p.i. (27.7 ± 3.1 % ID/g) (Fig. 2, Table 3). Activity elimination from blood is rapid with a blood activity concentration of 0.3 ± 0.04 %ID/g 60 min p.i. This results in low non-liver organ uptake. Highest uptake in non-target organs could be found in the kidneys 10 min p.i. (8.2 ± 0.5 % ID/g). However, even there the liver to organ ratio is 3.5 10 min p.i. increasing to 11.4 60 min p.i. Most favourable liver-to-organ ratios were reached after 60 min p.i. in almost all organs including blood, spleen, pancreas, heart, lung and muscle demonstrating the described lack of ASGR1 expression in non-liver tissue and the good pharmacokinetic properties of this new compound (Fig. 3, Table 4). The rapid reduction of activity uptake in kidneys indicate fast renal excretion of the tracer. The only organ with increasing activity concentration over time is the intestine reaching 3.0± 0,4 % ID/g 60 min p.i.

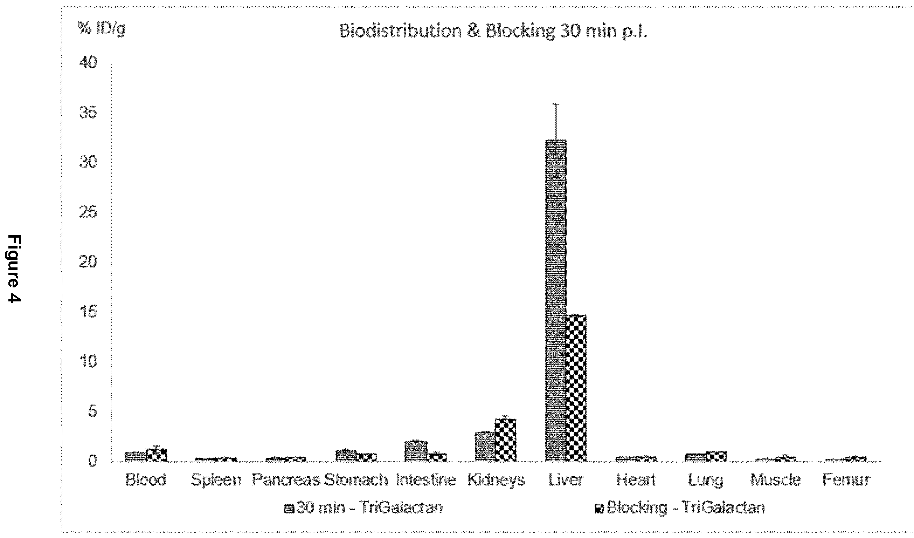

Blocking experiments were carried out co-injecting a high excess of D-Galactose and examining the mice 30 min p.i. (Fig. 4). A significant reduction in activity accumulation in the liver can be found (32.2 ± 3.7 %ID/g vs. 13.9 ± 1.0 %ID/g) whereas in most non-target organs comparable activity accumulation is observed. The only other organs with an activity reduction are stomach and intestine where uptake might be effected due to elimination of the high galactose amount and not due to specific interaction with the tracer.

The reference compound ^{99m}Tc-GSA showed higher liver uptake 10 min p.i. (58.1 ± 6.7 % ID/g,) but declines more significantly over the observation period of 60 min. (46.3 ± 5.9 % ID/g) (Fig. 2, Table 3). In contrast to [⁶⁸Ga]Ga-NODAGA-TriGalactan elimination from the blood pool is much slower and activity concentration in kidneys remains almost stable over the observation period of 60 min. Again, highest non-target uptake was found in the kidneys. The increase of activity in the intestine is also higher compared to the new tracer resulting in 9.8 ± 1.6 %ID/g vs. 3.0 ± 0.4 %ID/g 60 min p.i. In general activity concentration in observed organs and tissue is higher as found for [⁶⁸Ga]Ga-NODAGA-TriGalactan resulting for the later time points in inferior liver-to-organ ratios (Table 4).

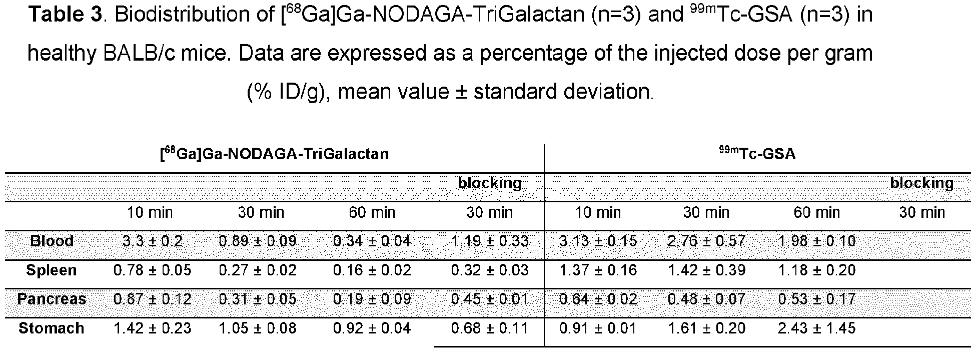

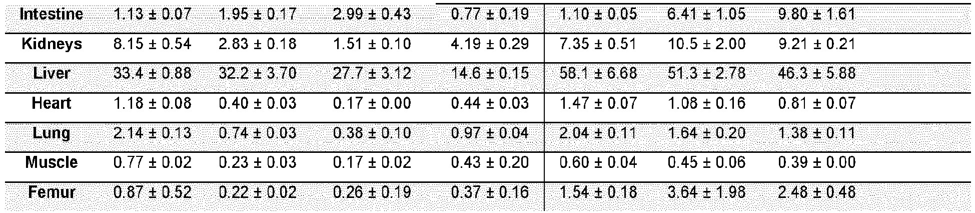

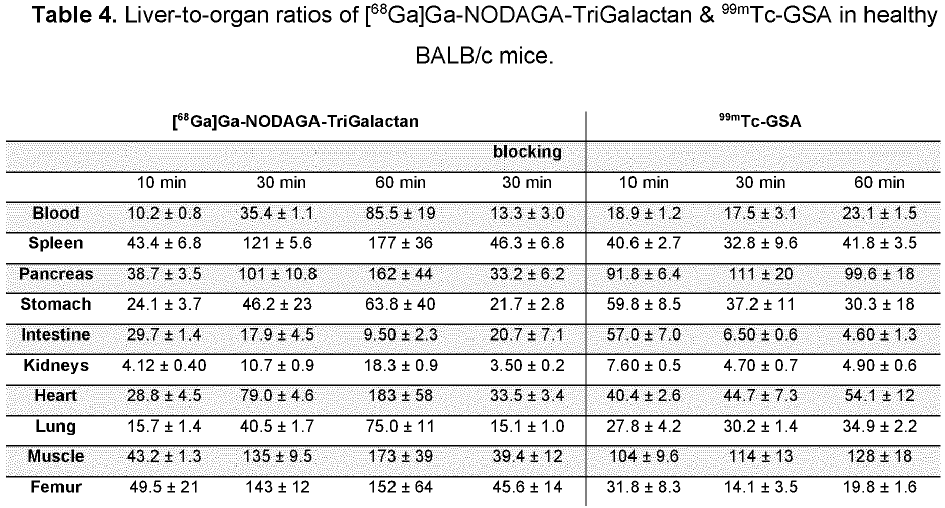

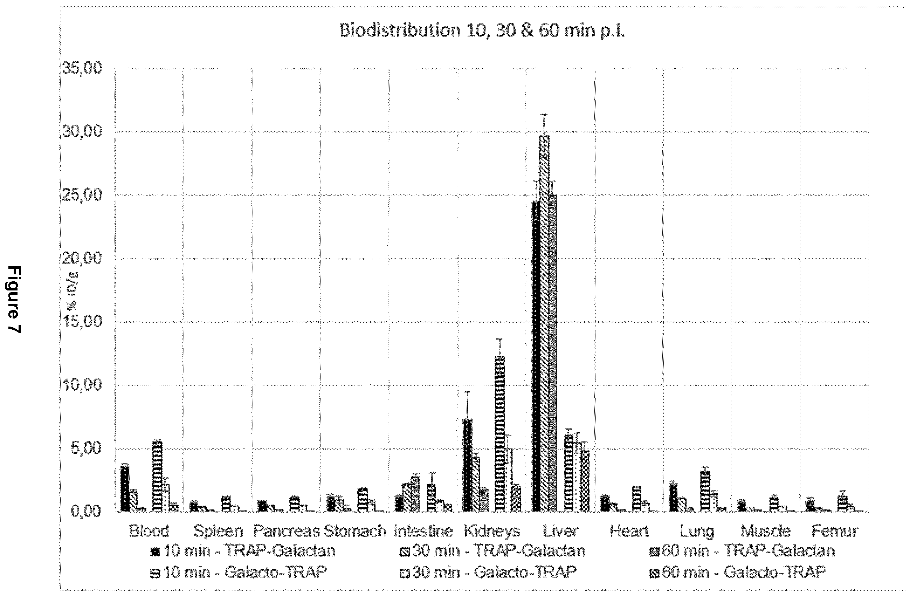

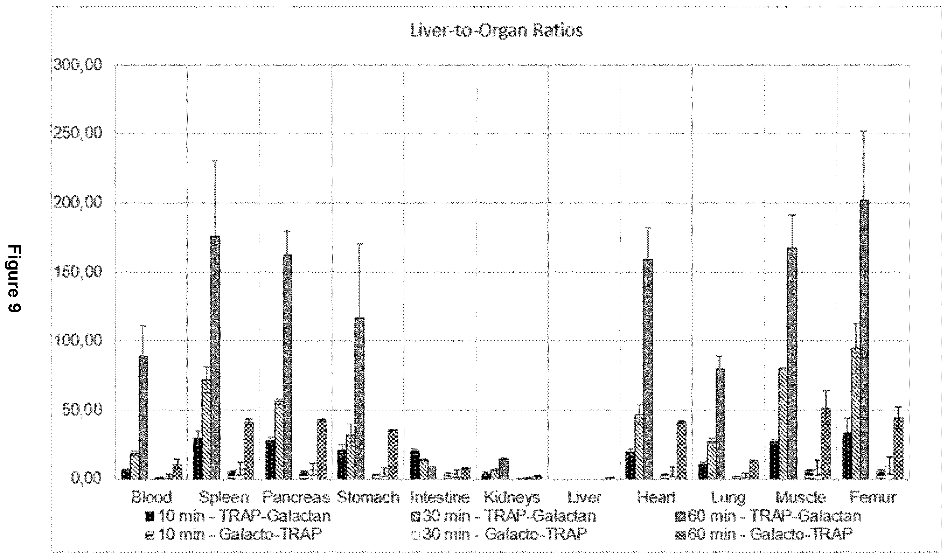

Biodistribution studies for [⁶⁸Ga]Ga-TRAP-Galactan showed high liver uptake starting from (24.6 ± 1.6 % ID/g) at 10 min p.i. and reaching its maximum 30 min p.i. (29.7 ± 1.7 % ID/g) (Fig. 7, Table 5). Activity elimination from blood is rapid with a blood activity concentration of 0.3 ± 0.09 %ID/g 60 min p.i. This results in low non-liver organ uptake. Highest uptake in non-target organs could be found in the kidneys 10 min p.i. (7.4 ± 2.1 % ID/g). However, even there the liver to organ ratio is 3.7 10 min p.i. increasing to 15 60 min p.i. Most favourable liver-to-organ ratios were reached after 60 min p.i. in almost all organs including blood, spleen, pancreas, heart, lung and muscle demonstrating the described lack of ASGR1 expression in non-liver tissue and the good pharmacokinetic properties of this new compound (Fig. 9, Table 6). The rapid reduction of activity uptake in kidneys indicate fast renal excretion of the tracer. The only organ with increasing activity concentration over time is the intestine reaching 2.8 ± 0.2 % ID/g 60 min p.i.

Biodistribution studies for [⁶⁸Ga]Ga-Galacto-TRAP revealed moderate liver uptake peaking at 10 min p.I. (6.1 ± 0.5 % ID/g) and declining to a value of 5.0 ± 0.6 % ID/g 60 min p.I. Highest non-target uptake was found in the kidneys (12.2 ± 1.4 %ID/g) and bloodpool (5.6 ± 0.2) 10 min p.I. Liver-to-organ ratios were generally lower than that for TRAP-Galactan, but still reached an optimum 60 min p.I. in spleen, pancreas, stomach, heart, lung, muscle and femur.

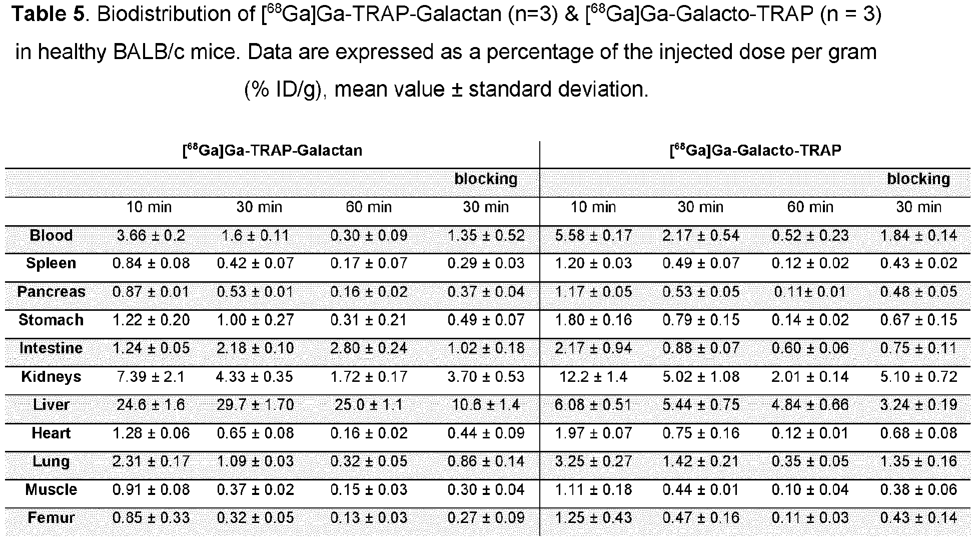

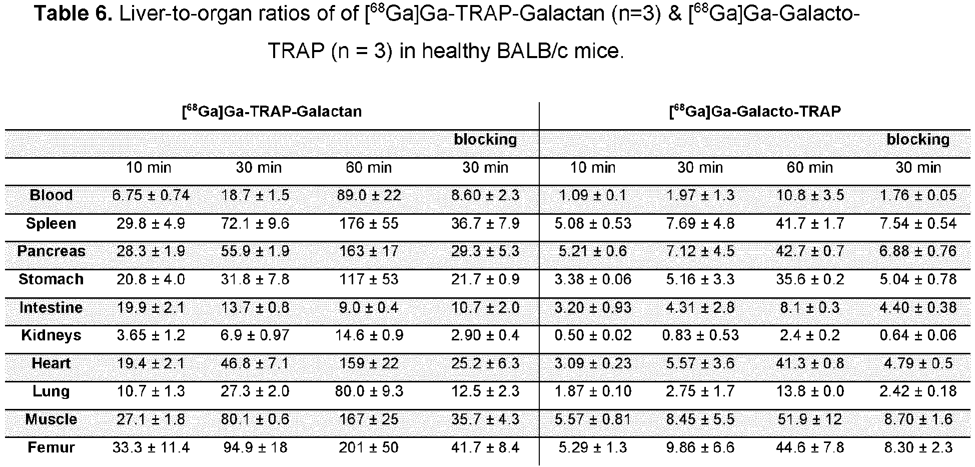

Blocking experiments for TRAP-Galactan and Galacto-TRAP were carried out co-injecting a high excess of D-Galactose and examining the mice 30 min p.i. (Fig. 9, Tables 5 and 6). A significant reduction in activity accumulation in the liver can be found for both tracers (10.6 ± 1.4 %ID/g and 3.24 ± 0.2 %ID/g) whereas in most non-target organs comparable activity accumulation is observed.

## Claims

1. A compound of formula (I), or a pharmaceutically acceptable salt thereof, 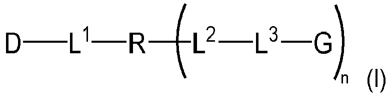 wherein
D is a chelating group or a prosthetic group or a [¹⁸F]F accepting group;
-L¹-R is absent or
L¹ is a linker selected from the group consisting of *-N(H)-(CH₂)ₓ-C(O)-, aspartic acid, glutamic acid, asparagine, glutamine, lysine, *-N(H)-(CH₂-CH₂-O)_{y}-(CH₂)_{z}-C(O)-, 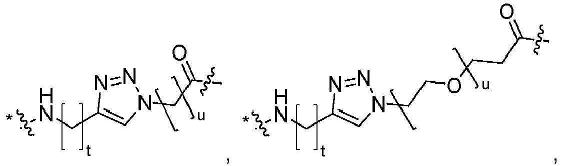 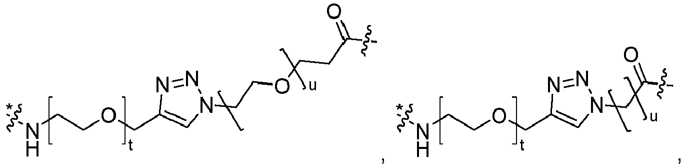 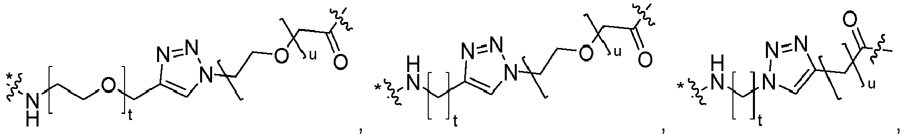 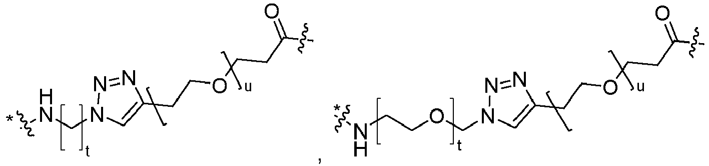 and 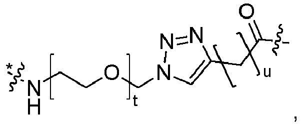 wherein* designates the attachment point to D, and
R is a multivalent group of the following formula 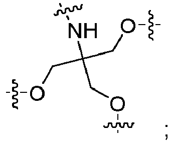
L² is a linker selected from the group consisting of 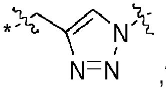 *-CH₂-C(O)-NH-, *-CH₂- 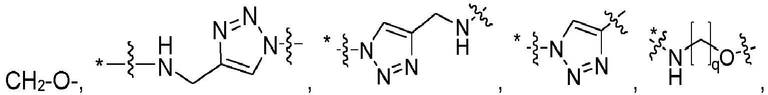 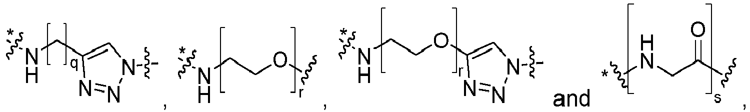 wherein * designates
i) the attachment point to D, if -L¹-R is absent, or
ii) the attachment point to R, if -L¹-R is present;
L³ is absent or a linker selected from the group consisting of ^{#}-(CH₂-CH₃-O)ₘ-CH₂-CH₂-, ^{#}-(CH₂)ₘ-, and 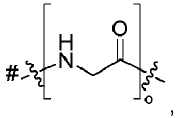 wherein ^{#} designates the attachment point to L²;
G is Gal, GalNAc, or Lac;
m is independently selected from 1-36;
n is 3 or 4, with the proviso that n is 3 if -L¹-R is present;
o is 3-6;
q is independently selected from 1-16;
r is independently selected from 1-36;
s is 1-10;
t is independently selected from 1-8;
u is independently selected from 1-8;
x is 1-16;
y is 1-36; and
z is 1-2.

2. The compound according to claim 1, or a pharmaceutically salt thereof, wherein D is a chelating group selected from the group consisting of NODAGA, NOTA, DOTAGA, DO3A, HBED, NCS-DTPA, p-NCS-Bz-DFO, FusC, DafC, CNAAZTA, NOPO, TRAP, DOTPI, DOTAZA, sacrophagine-based chelators, and TE2A derivatives such as CB-TE2A.

3. The compound according to claim 1 or 2, or a pharmaceutically salt thereof, wherein
-L¹-R is present and
D is a chelating group selected from the group consisting of 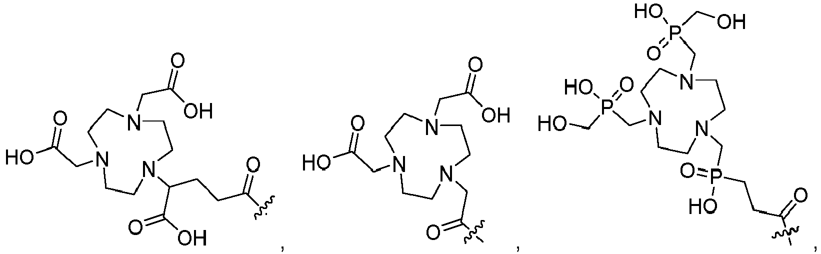 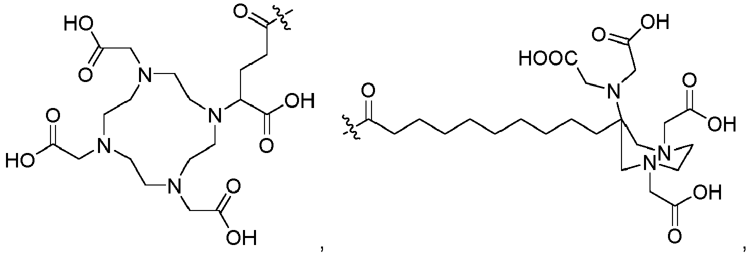 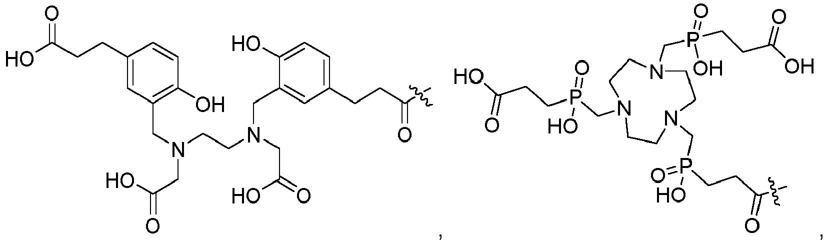 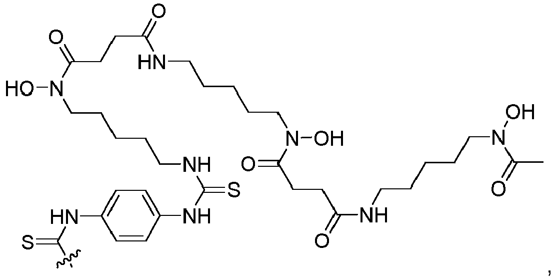 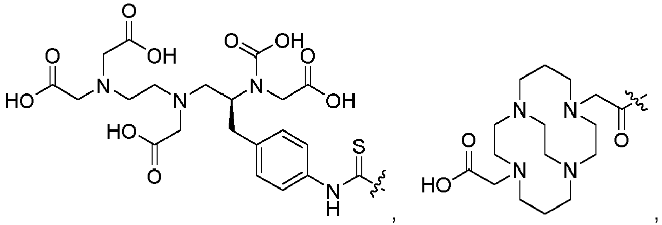 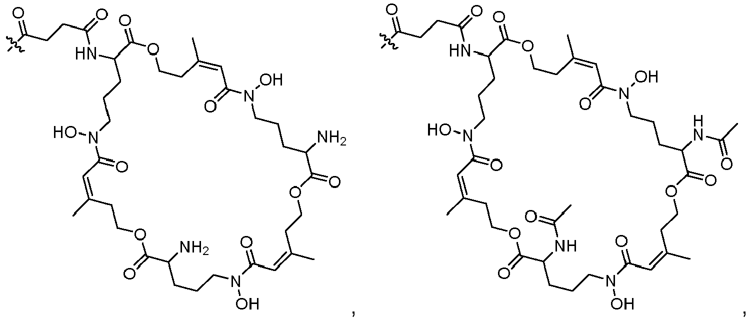 and

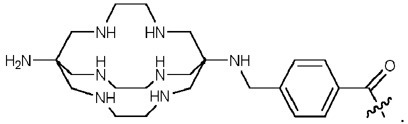

4. The compound according to claim 3, or a pharmaceutically acceptable salt thereof, wherein
L¹ is *-N(H)-(CH₂)ₓ₋C(O)-, and * designates the attachment point to D; and/or
L² is 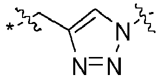 and * designates the attachment point to R; and/or
L³ is ^{#}-(CH₂-CH₂-O)ₘ-CH₂CH₂-, and ^{#} designates the attachment point to L².

5. The compound according to claim 3 or 4, or a pharmaceutically acceptable salt thereof, wherein the compound is

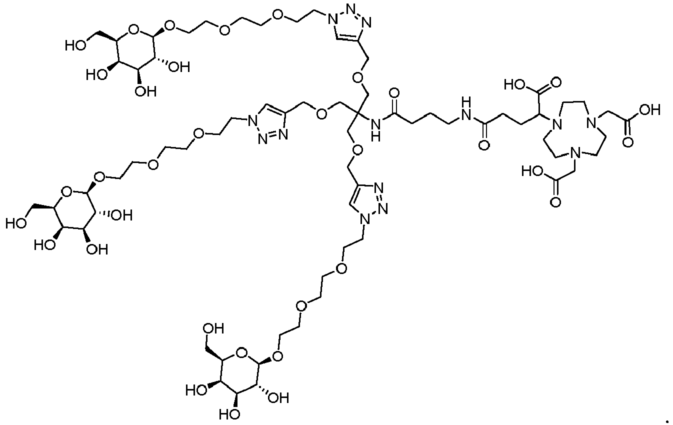

6. The compound according to claim 1 or 2, or a pharmaceutically acceptable salt thereof, wherein -L¹-R is absent and
D is chelating group selected from the group consisting of 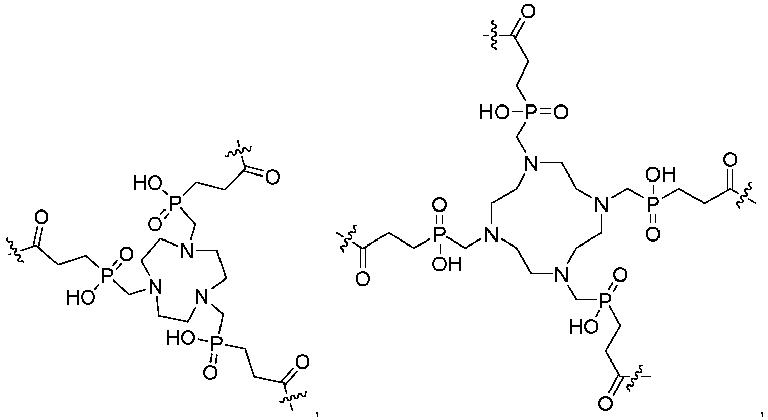 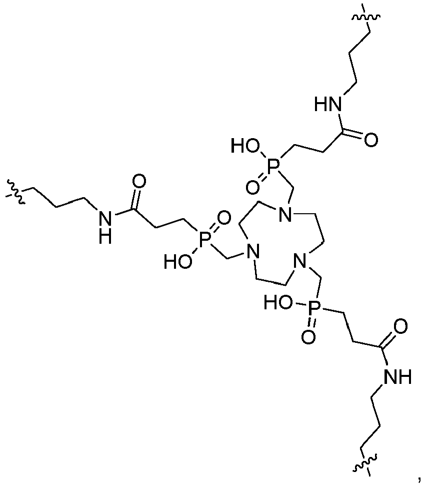 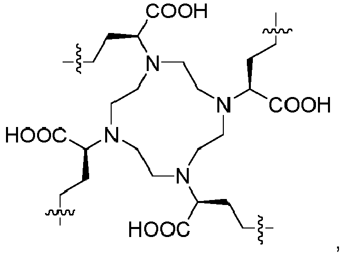 and

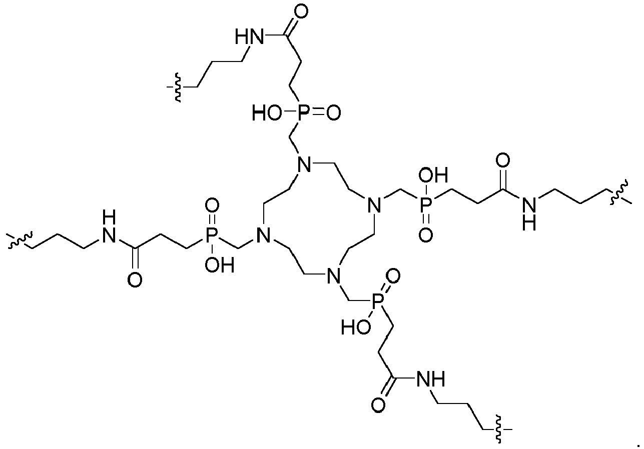

7. The compound according to claim 6, or a pharmaceutically acceptable salt thereof, wherein
L² is 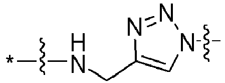 and * designates the attachment point to D; and/or
L³ is absent or L³ is ^{#}-(CH₂-CH₂-O)ₘ-CH₂CH₂-, and ^{#} designates the attachment point to L².

8. The compound according to claim 6 or 7, or a pharmaceutically acceptable salt thereof, wherein the compound is

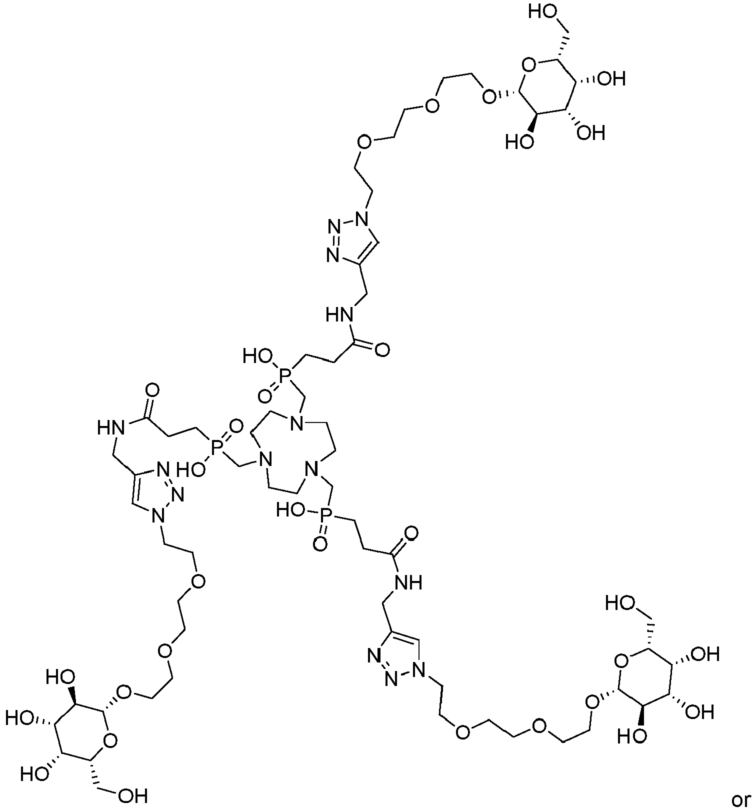

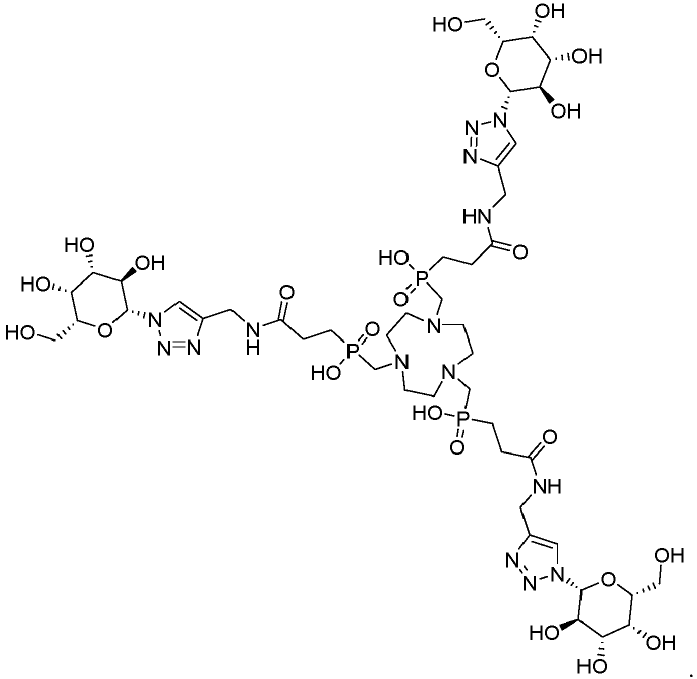

9. The compound according to any one of claims 1-8, or a pharmaceutically acceptable salt thereof, wherein D chelates [⁶⁸Ga]Ga, [¹⁸F]AlF, [⁸⁹Zr]Zr, [⁶⁴Cu]Cu or [⁸⁶Y]Y.

10. A method of producing the compound according to any one of claims 1-9, comprising synthesizing the compound.

11. A pharmaceutical or diagnostic composition comprising the compound according to any one of claims 1-9 and at least one additive.

12. A method of imaging hepatocytes, comprising a) contacting the hepatocyte with a compound according to any one of claims 1-9, and b) visualizing the compound that is in contact with the hepatocyte.

13. A method of determining functional hepatic reserve, comprising a) contacting the liver with the compound according to any one of claims 1-9, and b) visualizing the compound that is in contact with the liver.

14. The method according to claim 12 or 13, wherein the compound is visualized by positron emission tomography (PET).

15. Use of the compound according to any one of claims 1-9 for imaging hepatocytes.

16. Use of the compound according to any one of claims 1-9 for determining functional hepatic reserve.
